# NEW EUROPEAN PATENT SPECIFICATION

(11) **EP 3 781 223 B2**
(45) Date of publication and mention of the opposition decision: **11.06.2025**
(45) Mention of the grant of the patent: 27.07.2022
(21) Application number: 18786352.7
(22) Date of filing: 18.10.2018
(51) Int. Cl.: A61M 1/00, A61F 13/00, A61F 13/02, A61M 39/24

(54) **WOUND CARE SYSTEM FOR NEGATIVE PRESSURE THERAPY**
WUNDPFLEGESYSTEM FÜR DRUCKREDUZIERTEN THERAPIE
SYSTÈME DE SOIN DES PLAIES TRAITEMENT À PRESSION RÉDUITE

(30) Priority: 18.04.2018 WO PCT/EP2018/059913
(43) Date of publication of application: 24.02.2021
(73) Proprietor: ConvaTec Limited, Deeside Industrial Park Deeside Flintshire CH5 2NU (GB)
(72) Inventor: HENTRICH, Axel, 1060 Wien (AT); SIX-SUDERA, Edda, 1230 Wien (AT); BRÜGGEMANN, Heinrich-Maria, 89597 Munderkingen (DE); SCHRÖDER-DUBOIS, Ludwig, 99084 Erfurt (DE)
(74) Representative: D Young & Co LLP
(86) International application number: PCT/EP2018/078594
(87) International publication number: WO 2019/201460

(56) References cited:
- WO-A1-2012/151359
- WO-A1-2015/123340
- WO-A2-2009/124125
- FR-A- 1 163 907
- GB-A- 2 525 658
- US-A1- 2010 137 775
- US-A1- 2012 316 538

## Description

The invention relates to a wound care system according to claim 1.

It has been shown that not only the healing of chronic wounds, but also the healing of acute wounds can be assisted by applying reduced pressure to these wounds. Corresponding wound care systems are specified, for example, in EP-A-2822613. With the wound care systems described in this specification the wound space is filled with an absorbent filling material. A gas-tight but water vapour permeable covering device in the form of a covering film, which may comprise, for example, a polyurethane polymer, in particular an aromatic polyurethane polymer, is attached to the side of the filling material facing away from the base of the wound, and is fastened, fluid-tight, to the skin surrounding the wound. For this purpose the boundary surface of the covering film facing the skin can be provided, at least in some areas, with at least one adhesive agent.

In the known wound care system a suction port is attached to the side of the covering device facing away from the filling material, by means of which the wound space can be connected to a pump designed to generate reduced pressure. In wound care systems of this type there is disposed between the suction port and the pump a tube which allows the patient to have a certain degree of mobility during the reduced pressure therapy.

The aim of current developments in the field of reduced pressure therapy is to provide portable systems in which the pumps are designed to be smaller and lighter. This applies in particular to the treatment of slightly to moderately exuding wounds. For the treatment of these wounds within the framework of reduced pressure therapy, purely mechanically operated pumps can be used which are connected by a tube to a modern wound dressing. This wound dressing may comprise a covering device and a wound filling material. In order to avoid the use of an exudate canister, which is generally otherwise required within the framework of reduced pressure therapy, an absorbent material, such as for example a superabsorber, may be used in the region of the filling material. The aim of the development of corresponding systems is to avoid burdening the patient when carrying the latter and to restrict his or her freedom of movement as little as possible. However, it has been shown that within the framework of the use of corresponding wound care systems there have in many cases only been poor therapy results.

Wound care systems according to the pre-characterizing portion of claim 1 are disclosed in US 2012/316538 A1, WO 2015/123340 A1, FR 1163907 A, US 2012/238971 A1 and US 2010/137775 A1.

In view of the problems in the prior art, the object underlying the invention is to provide a wound care system of the type specified at the start which improves the mobility of a patient, increases the comfort of the patient and the ease of use, reduces the size of the value coupled to the system and with which the desired therapy result can be achieved with improved reliability.

According to the invention this object is achieved by a further development of the known wound care systems as specified in the characterizing portion of claim 1.

This invention is based on the finding that the failure of the therapy observed with the conventional wound care systems is in many cases explained by the fact that unnoticed leaks occur in the region of the connection tubes between the suction port of the wound covering and the pump. Due to these leakages, gas penetrates into the wound space and the pressure in the wound space is increased. Then reduced pressure therapy no longer takes place.

Within the framework of this invention it has been discovered that these deficiencies can be eliminated by using pumps with a modular structure. A pump designed to generate reduced pressure generally comprises at least two valves, one of which enables the discharge of gases from the volume to be evacuated, in this case therefore the wound space, but counters the entry of gases into the volume to be evacuated, whereas the other valve enables the discharge of gases sucked out of the volume to be evacuated into the surrounding area.

In wound care systems according to the invention only one of these valve systems is assigned directly to the covering device or to the wound space, whereas the other valve system can, if so required, be coupled detachably thereto in order to generate the desired reduced pressure in the wound space. After the corresponding suction procedure the suction device can be detached from the valve system, which subsequently counters the entry of ambient air into the wound space and so maintains the reduced pressure in the wound space.

The invention has been explained above in connection with the use of the valve system assigned to the covering device as a component part of a pump that can be used to produce reduced pressure. However, the invention can also be used in combination with conventional pumps, the suction port of which is coupled to the valve system assigned to the covering device.

With wound care systems according to the invention the reduced pressure can be maintained over a period of a number of hours despite the uptake of exudate, possibly with the use of a wound material containing an absorbent material, such as for example a superabsorber. The suction device itself does not necessarily have to be carried along over this period of time. In any case, it is not necessary to couple the suction device itself to the covering device with the aid of troublesome tubes. If so required, it can be coupled to the valve device assigned to the covering device. Small and light suction devices, possibly in the form of small mechanical or electromechanical pumps which the patient may, if so required, carry with him or her, may be used here. He/she may, for example, carry them discretely in garment pockets.

Within the framework of the invention it is also conceivable to integrate the valve system assigned to the covering device into the covering device itself. However, it has proven to be particularly advantageous if the valve system is coupled to the covering device by means of a short fluid line, referred to in the following as a tube, without any restriction to particular geometries or materials. In this embodiment the valve system can be coupled to the suction device without interfering with the wound itself. On the other hand, in this embodiment of the invention it is not necessary to provide a long tube between the valve system and the covering device. Purely by means of a short fluid line or a short tube, which is only slightly prone to defects, and with a length of only 20 cm or less, in particular only 10 cm or less, but more than 3 cm, in particular more than 5 cm, between the covering device and the valve system, sufficient separation of the pumping process from the wound space can be ensured. In some applications, such as for example in a combination with compression therapy, longer fluid lines or tubes may, however, also be used. Tubes with a length of 50 cm or more, in particular of 60 cm or more, are conceivable. The length of the tubes may be based upon the length of a compression sock that may be used. In any case, with wound care systems according to the invention it is not necessary to permanently maintain a connection between the suction device and the wound space, as a result of which mobility is improved in all cases.

The valve system has a valve body with a valve chamber serving to receive gases from the wound space, that is preferably expandable or can be compressed so as to release gases held within it. The use of corresponding valve systems enables the use of simple mechanical suction devices which, designed as simple piston or also bellows pumps, can easily be kept in a bag and can be operated without any electric current, but they may also be used in conjunction with electromechanical pumps.

A further advantage of a valve system having a valve chamber is seen in the fact that the valve chamber provides a supplemental volume which increases the overall volume in which a reduced pressure is to be generated.

This overall volume comprises the volume of the wound space, the volume of the tube connecting the valve chamber with wound space and the volume of the valve chamber itself. Thus, in order to increase the pressure within the wound space it is necessary to introduce gas into the overall volume, i. e. also into the valve chamber. The additional volume of the valve chamber helps to preserve the reduced pressure within the wound space over an extended period of time within an acceptable range.

The valve system of a wound care system according to the invention, in particular its valve chamber, may comprise a one-way valve designed to discharge fluids, in particular gases, from the wound space. Within the framework of this description and the claims, the expression one-way valve includes in particular check valves.

According to this invention the one-way valve is made in the form of a film valve that is described in more detail below.

In the expanded state the valve chamber is approximately lens-shaped in form and is delimited by two film-like limiting elements connected, gas-tight, to one another in the region of circumferential edges of the lens, both of which are provided with at least one valve slot which, on the one hand, allows gasses to pass out of the valve chamber, but on the other hand counters the entry of gases from outside into the valve chamber. The advantage when using valve chambers, in which both limiting elements are provided with one-way valves, can be seen in that when coupling the valve system to the suction device, one does not have to pay heed to the alignment of the preferably lens-shaped valve chamber. As a result, operation thereof is facilitated, and operating errors are largely ruled out.

Leaks in the region of the valve body can be more reliably avoided if at least one limiting element of the valve body has at least one reinforcement layer provided with a recess in the region of the at least one valve slot.

If the valve system is coupled to the covering device by means of a tube, this tube advantageously leads into the valve chamber. The gases discharged from the wound space can thus be conveyed directly into the valve chamber from which they can be discharged via the one-way valve into the surrounding area with the aid of the suction device.

The valve system of the invention comprises a film valve as defined in claim 1. Film valves are described, for example, in CN 201325638, WO 2016/040029 A1 and DE 10 2004 002 843 A1. This type of film valve essentially comprises at least two, preferably three film layers, two of which are provided with through holes lying one over the other. An outer film layer covers the generally circular through hole of the other film layer. This film layer lies over the middle film layer, slot-like through holes being able to be provided in the contact area, but not above. The film layer provided with the slot-like through holes is connected to the middle film layer, forming a seal, outside of the circular through holes. Within the sealing bond, both films are connected to one another, either by static forces or by weak adhesive forces. If reduced pressure is applied to the layer of film provided with slot-like through holes and facing away from the middle film layer, this film layer is lifted from the middle film layer so that air penetrates through the generally circular through holes of the other film layer into the space between the middle film layer and the film layer provided with the slots and can escape through the slots.

In the structure that has been described the middle film layer has a stabilising character. If no great demands are made of the stability and/or the film layer facing away from the film layer provided with the slot-like through holes has sufficient stability, a film valve may also have only two film layers, one of which has a substantially circular through hole, and the other is provided with the slot-like through holes as described.

In this way, a very thin one-way valve is provided, by means of which evacuation of a space on the boundary surface of the middle layer facing away from the layer provided with the slots can be brought about by applying reduced pressure to the outer surface of the film layer provided with slots facing away from the middle film layer.

If the covering device is provided with a corresponding film valve, the suction device can be placed directly on the covering device. Mechanical, electromechanical, hydraulic and/or pneumatic pumps and suction devices can be used here.

As already explained above, the film-like, deformable and water vapour permeable covering device of wound care systems according to the invention is provided on areas or sections of one side with an adhesive agent designed to fix the covering device to the skin. Here, the adhesive agent may run around the entire wound space along an edge of the covering device. If the valve system is coupled to the covering device by means of a tube, the tube can be attached to the boundary surface of the covering provided with the adhesive agent so that it comes to rest between the covering device and the skin on the skin surrounding the wound after attaching the covering device. In this embodiment of the invention it must be ensured that no leaks occur between the skin and the covering device.

Within the framework of the invention it has proven to be particularly preferable if the tube passes through the covering device and/or leads, forming a seal, into an opening passing through the covering device. In this embodiment of the invention, the assembly consisting of the covering device and the tube or fluid line can be pre-fabricated. In this way it can be particularly reliably ensured that leaks do not occur in the region of the cross-over between the tube and the covering device. In addition or alternatively, the covering device may have a suction port designed to couple the fluid line, as is known, for example, from EP-A-2822613.

In particular, in such wound care systems, in which the valve system is coupled by means of a fluid line to the covering device, it has proven to be advantageous, in order to prevent damage to the valve system, if the valve system has an at least partially rigid housing which preferably at least partially delimits a valve chamber, providing the additional volume, as explained above and which is preferably provided with a one-way valve, in particular a film valve. The housing may at least partially enclose a valve chamber and have a through hole which is covered by the one-way valve, in particular the film valve, and by means of which the valve chamber and the wound space connected fluidically thereto can be subjected to reduced pressure. If the housing is approximately in the form of a cylinder, in particular a circular cylinder, the through hole may be provided in a front wall or end face of the cylinder.

According to another aspect of the invention, a wound care system for reduced pressure therapy with a covering device that can be fixed to the skin surrounding a wound and serving to produce a closed wound space containing the wound is essentially characterised in that an indicator device that can be operated to display the reduced pressure status in the wound space is assigned to the covering device.

This aspect of the invention is based on the finding that the therapy failures occasionally observed with conventional wound care systems for reduced pressure therapy may also be due to the fact that the increase in pressure within the wound space cannot be determined at all. This deficiency can be eliminated by the wound care system being additionally provided with an indicator device which informs the patient, the nursing staff or the doctor of the reduced pressure status in the wound space. If it is determined by reading the indicator device that an increase in pressure is taking place in the wound space, this deficiency can be eliminated immediately. Similarly to the valve system, within the framework of the invention the indicator device can also be integrated into the covering device. In terms of a modular structure it has, however, proven to be particularly advantageous if the indicator device is coupled by means of a tube or a fluid line to the covering device. Here, the tube, similarly to the fluid line connecting the valve system to the covering device, can be attached to the boundary surface of the covering device provided with the adhesive agent and/or pass through the cover and/or lead, forming a seal, into an opening passing through the covering device and/or be coupled to a connection or port of the covering device that can also be operated as a suction port.

In a particularly preferred embodiment of the invention the indicator device is integrated into the valve system, in particular into the valve chamber. For this purpose, at least one limiting element of the valve chamber, and optionally at least one reinforcing layer, is advantageously made to be transparent, at least in sections.

The indicator device may be made particularly simple in terms of structure if it has two indicator layers spaced apart from one another and running in particular approximately parallel to one another, which can be compressed, at least in some areas, against a pre-tensioning force so as to reduce the spacing. When the pressure between the indicator layers corresponds to the pressure in the wound space, the pre-tensioning force can be overcome by the pressure difference between atmospheric pressure and the reduced pressure in the wound space. This means that the compression of the indicator layers indicates sufficient reduced pressure in the wound space if the pre-tensioning force is set appropriately. In order to provide the pre-tensioning force, at least one in particular elastically deformable spacer layer disposed between the indicator layers and that can be compressed by overcoming a pre-determined pre-tensioning force, can be provided. It has proven to be particularly advantageous if the spacer layer has at least one window so that the space between the indicator layers is not filled by the spacer layer in the region of the window and can be used to give a visual representation of a changed distance between the indicator layers.

With a view to the desired monitoring of the reduced pressure in the wound space, it has proven to be advantageous if the tube connecting the indicator device to the wound space leads into an indicator space formed between the indicator layers, and preferably at least partially passes through the spacer layer. In this way one achieves correspondence between the pressure in the indicator space and the pressure in the wound space, and the pre-tensioning force that is set can be overcome by the pressure difference between the reduced pressure in the wound space and the atmospheric pressure.

In particular in the latterly described embodiment of the invention, the monitoring of the pressure in the wound space can take place particularly easily if a boundary surface of one indicator layer facing the other indicator layer has profiling or graphical representation which can be seen when the distance between the indicator layers is reduced, brought about by the pressure difference between atmospheric pressure and the reduced pressure in the wound space.

As with a valve system coupled to the wound space by means of a fluid line, an indicator device, in particular an indicator device coupled to the wound space by means of a fluid line, can also be effectively protected from damage if it has an at least partially rigid housing. Two chambers separated from one another by a gas-tight and deformable separating device can be formed in the housing, of which a first chamber can preferably be connected to the wound space by means of a fluid line, thereby providing a further additional volume, and of which a second chamber can preferably be connected to the surrounding area by means of a recess in the housing. The moveable and/or deformable separating device that separates the two chambers from one another can be realised by means of a flexible membrane or by a piston with a sealing ring resting against an inner chamber wall. As soon as reduced pressure is applied to the first chamber, and so also to the wound space associated with it, suction is produced which leads to movement or deformation of the separating device. This movement or deformation is made possible because pressure equalisation can take place in the second chamber. The application of reduced pressure to the first chamber therefore leads to a reduction of the volume of the first chamber and to an increase of the volume of the second chamber.

In one preferred embodiment of the invention a pre-tensioning device preferably comprising a compression spring is accommodated in the first chamber, by means of which pre-tensioning device the separating device, preferably made in the form of a deformable membrane, is held in an initial position, and against the pre-tensioning force of which the separating device is moved to a final position when reduced pressure is applied to the first chamber. By means of this structure it is ensured that a structure change takes place within the housing if reduced pressure is applied to the first chamber which, upon equalisation of the reduced pressure or upon application of normal pressure to the first chamber, is reversed again by the effect of the pre-tensioning device. This structure change may be recorded visually, acoustically and/or electronically and be used to monitor the reduced pressure status.

In terms of optical monitoring of the reduced pressure status, it has proven to be particularly advantageous if the separating device, in particular the deformable membrane used as the separating device, comes at least partially into contact with a preferably plane inner boundary surface of the preferably cylindrical, in particular circular cylindrical housing, upon reaching the end positions. The positioning of the separating device against the inner boundary surface of the chamber can be particularly reliably recorded visually. When using a cylindrical, in particular a circular cylindrical housing, each of the chambers is delimited by one of the front surfaces or end faces of the housing, in each front surface a recess being able to be formed which on the one hand enables pressure equalisation (second chamber), and on the other hand enables a fluidic connection to the wound space.

In a preferred embodiment of the invention the housing is at least partially built from a semi-transparent plastic so that in the reduced pressure-free state only one-colour housing walls can be seen. In addition or as an alternative to the through hole in a front surface, the first chamber can also have an additional port or connection for establishing a connection to the wound space. In any case, by means of a recess in a front surface of the housing delimiting the first chamber, a film valve can be provided by means of which the first chamber, and so also the wound space connected fluidically to the latter can be subjected to reduced pressure. In the reduced pressure-free state the separating device in the second chamber lies on a front surface of the housing delimiting this second chamber. If the first chamber, and so also the wound space, is subjected to reduced pressure via the film valve, the volume of gas in the first chamber can be reduced by deformation or movement of the separating device, as a result of which a compression spring accommodated within the first chamber and which pushes the separating device into the initial position is tensioned. Under normal pressure the separating device, and optionally an image thereof provided on a boundary surface facing the housing wall delimiting the first chamber is pushed away from the housing wall. By tensioning the compression spring upon applying reduced pressure to the first chamber, the image provided on the corresponding boundary surface of the separating device is pressed from the inside against the housing wall. The image thus becomes visible through the semi-transparent housing wall and displays the reduced pressure status qualitatively.

At the same time as the volume reduction in the first chamber, an equalising volume of air can flow through the corresponding hole into the second chamber. The reduced pressure from which the reduced pressure status can be visually identified qualitatively can be set by the choice of spring force and the dimensions of the housing interior. When the pressure rises in the first chamber or when the reduced pressure drops in the first chamber the pre-tensioning device pushes the separating device back into its initial position, and so also the image back into its original position. On the other hand, the image can no longer be seen from a specific reduced pressure status.

With a modification of the indicator device provided with a rigid housing, two sleeves that engage in one another and that can be displaced relative to one another against the pre-tensioning force of the pre-tensioning device can be disposed in one of the chambers, preferably in the first chamber, one of which sleeves is coupled to a housing wall, and the other of which is coupled to the separating device. If the separating device is moved or deformed by applying reduced pressure to the first chamber, the positional relationship between the sleeves that engage in one another changes. In this way the overlapping region of the sleeves that engage in one another is also changed. The dimensions can be chosen so that an outer sleeve hardly covers an inner sleeve in an initial position of the separating device, but in the final position reached by applying reduced pressure it totally covers the inner sleeve. By different colouring of the sleeves that engage in one another, the reduced pressure status can thus be visually monitored qualitatively. For example, the outer sleeve, which completely overlaps the inner sleeve in the final position of the separating device, can be coloured green, whereas the inner sleeve, which is visible in the initial position of the separating device, can be coloured yellow or red.

As emerges from the above explanation, within the framework of the invention it has proven to be particularly advantageous if a housing wall delimiting the first chamber of the housing is provided with a one-way valve, in particular a film valve, by means of which reduced pressure can be applied to the first chamber, and so also the wound space fluidically connected thereto.

According to a further aspect of the invention the wound care system can have a chamber that is fluidically connected to the wound space and that is preferably substantially cylindrical, which chamber is delimited by a circumferential chamber jacket and has an indicator wall optionally forming a front wall or end face of the chamber and moveable between an initial position and a final position, the indicator wall being able to be moved from the initial position to the final position by applying reduced pressure to the chamber.

In a particularly preferred embodiment of the invention, the indicator wall is accommodated in the chamber to thereby prevent direct access to the indicator wall from the outside. In this embodiment of the invention, a rigid supporting member, preferably forming an end face or front wall of the chamber, may be provided which is adapted to abut with an abutting surface thereof against the indicator wall in the initial position thereof and which carries on its outer surface which faces away from the abutting surface a valve assembly, in particular a film valve, countering penetration of gases into the chamber and operable to suck gases out of the chamber.

In this embodiment of the invention, haptic control of the reduced pressure status within the chamber and thus within the wound space is made possible by sealingly attaching the supporting member to the circumferential chamber jacket by means of a deformable, in particular an elastically deformable, coupling member.

By using such a construction where the supporting member which may also form an end face or front wall of the chamber is coupled to the circumferential chamber jacket, it is possible that the supporting member is urged into the chamber by applying reduced pressure to the chamber via the valve system, e. g. the film valve, thanks to the provision of the deformable coupling member. Thus, the positional relationship between the supporting member and the circumferential chamber jacket depends on the reduced pressure status within the chamber to thereby offer haptic control of the reduced pressure status.

In a further preferred embodiment of the invention the indicator wall is urged into its final position by the supporting member when applying reduced pressure to the chamber to thereby effect the supporting member to be urged into the chamber contacting the indicator wall with its abutting surface. Movement of the rigid supporting member into the chamber may also effect automatic release of a suction device from the valve system to thereby automatically prevent further reduction of pressure in the valve chamber.

In this embodiment of the invention the wound care system may have just one chamber which at the same time performs the function of a valve chamber and the function of an indicator chamber. The moveable indicator wall or supporting member makes it possible to monitor the reduced pressure status haptically. Since the indicator wall or the supporting member delimits the chamber, i.e. it is disposed between the chamber itself and the surrounding area, the boundary surface of the indicator wall or the supporting member facing away from the chamber is easily accessible. It can be established by feeling whether the indicator wall or supporting member is in the initial position or in the final position. If it is established by haptic monitoring that the indicator wall is in the initial position, it can be concluded that the reduced pressure status in the chamber, and so also in the wound space, is no longer satisfactory, and gases must be sucked out of the wound space, possibly via the chamber, with the aid of an appropriate suction device. The chamber wall is preferably designed here so that it automatically adopts the initial position in the force-free state, i.e. when the same pressure conditions prevail on both sides of the chamber wall.

Haptic monitoring of the reduced pressure status is particularly easy if the indicator wall is curved outwards towards the space lying outside of the chamber and/or towards a space lying between the supporting member and the indicator wall in the initial position and/or is curved into the chamber in the final position. The automatic transition from the final position into the initial position can be realised particularly easily if the indicator wall has a correspondingly outwardly curved snap disc in the unstressed state, i.e. when the same pressure conditions prevail on both sides of the indicator wall. If the gases are to be sucked out of the wound space via the chamber, it has proven to be advantageous if a recess preferably positioned in the crest region of the curve and serving to apply reduced pressure to the chamber, and so also to the wound space, passes through the snap disc and optionally through the supporting member, where the recess in the supporting member is aligned to the recess in the indicator wall.

In terms of establishing a sealing connection between the chamber jacket and the indicator wall, it has proven to be advantageous if a circumferential edge region of the indicator wall is accommodated in an inner groove of the chamber jacket that is open towards the chamber. Advantageously, the indicator wall has circular edges here.

Trouble-free folding down of the indicator wall from the initial position into the final position can be achieved if, in the initial position and/or the final position of the indicator wall, a space is formed between the bottom of the inner groove and the edge region of the indicator wall into which the edge region of the indicator wall can pass during the folding process.

A sealing connection between the chamber jacket and the indicator wall is further assisted if edge regions of the walls of the inner groove coming into contact with the circumferential edge region of the indicator wall are formed at least partially from a flexible material so that these regions can nestle against the indicator wall in order to establish a sealing connection. The flexible regions of the groove wall can also be realised in the form of sealing rings inserted into the groove wall. However, it has proven to be particularly advantageous if the groove walls are overall formed in one piece and optionally have circumferential sealing ribs.

Within the framework of the invention it has proven to be particularly advantageous if a circumferential collar for the form-locking coupling of a suction head of a suction device is formed in the region of the cross-over between the indicator wall and the chamber jacket, the collar preferably forming a stop limiting the approach of the suction head to the recess passing through the snap disc and/or the supporting member carrying the film valve. By adapting the dimensions of the collar to the dimensions of the suction head and the position of the snap disc and/or the supporting member, it can thus be ensured that in the initial position of the snap disc the suction head can be moved sufficiently close to the opening passing through the snap disc or the supporting member so as to thus establish a sealing connection between the suction head and the recess passing through the snap disc and/or the supporting member. At the same time, by limiting the approach of the suction head to the recess passing through the snap disc and/or the supporting member, it is guaranteed that the sealing connection between the suction head and the film valve covering the recess passing through the snap disc and/or the supporting member is automatically released when, upon reaching a sufficient reduced pressure in the chamber, the snap disc folds down into the final position in which it is curved into the chamber. The suction head can then be released from the collar, unhindered by any pressure difference between the chamber and the surrounding area.

The collar can be made in the form of an annular protrusion extending the chamber jacket in the axial direction. However, it can also be made in the form of a setback between a front surface of the chamber jacket and the outer boundary surface of the snap disc.

The indicator wall can have a valve system covering the recess of the snap disc, preferably in the form of a film valve, serving to suck gases out of the chamber and countering the penetration of gases into the chamber. The valve system can be disposed on the boundary surface of the snap disc facing away from the chamber and/or on an outer surface of the rigid supporting member. By disposing the film valve on the rigid supporting member to thereby enabling sucking of gas out of the valve chamber via a recess within the rigid supporting member it is prevented that the film valve is deformed by a movement of the indicator wall between an initial position and a final position. Thus reliability of the system may be improved because deforming the film valve may provoke peeling off of the film valve. Thus, the use of a rigid supporting member for the film valve which helps to prevent deformation of the film valve is preferred.

In order to establish a fluid-conveying connection between the chamber and the wound space, a channel leading into the chamber can pass through the chamber jacket, to which channel a tube connecting the chamber to the wound space can be coupled.

In this embodiment of the invention, with a view to avoiding damage to the tube it has proven to be particularly advantageous if a circumferential outer groove for accommodating a tube connecting the chamber to the wound space is provided. The tube can preferably be wound up in the outer groove which is provided in the outer boundary surface of the chamber jacket facing away from the chamber. The channel which leads into the chamber, on its side facing away from the chamber leads into the outer groove. On its side facing away from the wound space the tube can lead into the channel or be at least partially accommodated in the channel. The required tube length can be set by winding up or unwinding the tube onto or from the circumferential outer groove. In an approach to avoid damage of the tube it is preferred that at least one transition region is provided between the groove and the channel which transition region has a guiding surface for the tube which tube preferably is at least in part accommodated in the channel and may terminate into the chamber. When the guiding surface on the one hand merges tangentially into the channel and on the other hand merges tangentially into the groove and preferably has a radius of curvature of 10 % or more, preferably 20 % or more of the radius of curvature of a bottom wall of the outer groove, folding or kinking of the tube in a region between the channel and the groove may be prevented.

In a particularly preferred embodiment of the invention two transition regions between the groove and the channel are provided which in combination form a funnel-shaped entry region which merges into the channel and makes it possible to wind up the tube in both directions without risking to kink or fold the tube at the transition between the channel and the circumferential groove.

The part of the tube that is not required is then accommodated, protected, in the outer groove. Undesired unwinding of the tube from the outer groove can be prevented if the latter has locking means for locking the tube emerging out of the outer groove.

These locking means can be realised by special shaping of the groove walls. In this connection it is conceivable for at least one groove wall to have on its side facing away from the bottom of the groove an indentation through which the tube can be moved out of the outer groove, the cross-over of the indentation to the adjacent parts of the groove wall countering a circumferential movement of the tube emerging out of the groove. Consequently, in the region of the indentation, which serves to guide the tube out of the groove, the groove has a larger width than outside of the indentation, the width in the region of the indentation may correspond approximately to the tube diameter. The tube must then be squeezed somewhat for the purpose of unwinding and/or winding up so as to thus be able to leave the region of the indentation. In this way locking of the tube can be achieved, which counters undesired unwinding of non-required tube sections out of the groove. In order to avoid squeezing of the tube it is also conceivable to make the groove walls at least partially of a flexible material. Then the tube can be moved out of the indentation by squeezing the groove walls. A recess can pass through at least one wall of the outer groove in order to discharge liquid from said recess. This has proven to be particularly worthwhile in cases in which the groove opening is closed by the tube wound up in the groove and can no longer be reached from every point of the groove interior and the wound care system can come into contact with moisture, for example when bathing.

It has already been explained above that haptic monitoring of the reduced pressure status is possible in this embodiment of the invention. Additional visual monitoring of the reduced pressure status is made possible with this embodiment of the invention if the chamber is delimited by an at least partially transparent monitoring wall on the side facing away from the indicator wall. Visual monitoring is facilitated if an indicator element coupled to the indicator wall is disposed in the chamber, which indicator element is moved towards the monitoring wall upon reaching the final position of the indicator wall and preferably comes into contact with the monitoring wall. In addition or alternatively, the indicator wall can be made to be deformable so that it rests against the chamber-side boundary surface of the indicator wall which is then in the final position when there is a desired reduced pressure in the chamber. The chamber-side boundary surface of the indicator wall may have a particular graphical configuration or profiling in order to facilitate monitoring of the reduced pressure status.

In one particularly preferred embodiment of the invention the boundary surface of the monitoring wall and/or of the chamber jacket facing away from the chamber is provided with an adhesive agent realised, for example, in the form of a hooked strip of a surface hook and loop fastener, which adhesive agent facilitates, for example, the attachment of the chamber to the boundary surface of the covering device facing away from the wound space.

In another preferred embodiment of the invention the chamber, together with the chamber jacket, is made to be substantially rotationally symmetrical, the chamber jacket being able to be made with particularly thick walls so that overall an appearance resembling a puck is produced. The wall thickness of the chamber jacket can be 30 % or more, preferably 50 % or more of the diameter of the chamber. The outside diameter of the chamber jacket can be 150 % or more, in particular 200 % or more, particularly preferably 250 % or more of the chamber height in the direction of the axis of rotation.

As explained herein before, in a particularly preferred embodiment of the invention the chamber wall is preferably designed such that it automatically adopts the initial position in the force-free state, i. e. when the same pressure conditions prevail on both sides of the chamber wall. In case an indicator wall is accommodated within the chamber and adapted to abut against an inner surface of the chamber wall, automatic transition of the chamber wall from its final position to its initial position may be assisted by the indicator wall. In case same pressure conditions prevail within the chamber and outside the chamber, no force counteracting automatic return of the indicator wall (snap disc) into its initial position is present such that the indicator wall automatically adopts its initial position.

In a further preferred embodiment of the invention signalling means are provided which are operable to provide a signal in the event where the indicator wall returns from its final position to its initial position. This signal may present an alert signal indicating that pressure within the indicator chamber or valve chamber and thus also within the wound space increased and it is necessary to apply the suction device in order to generate the desired reduced pressure.

Adequate indicator means may comprise an NFC (Near Field Communication) chip which may be coupled to an electronic device like a cell phone or smartphone. This enables wireless communication between the indicator means and the electronic device.

Within the scope of this invention it is further possible to integrate indicator means into fixing means to which the valve chamber or indicator chamber may be fixed detouchably. Coupling of the indicator wall to the fixing means may be effected mechanically or electronically, while the signal which indicates transition of the indicator wall from its final position to its initial position is produced within fixing means.

As explained herein before, signalling means may be coupled wirelessly with any electronic means which may be operable to produce an optical, haptic or acoustical alert signal in the event a signal is received from the signalling means. Such electronic means may be associated with suction means. In case signalling means are accommodated in separate fixing means it is also possible to transmit respective signals via Bluetooth to cell phone or smartphone which may have installed a corresponding app. In this case return of the indicator wall from its final position to its initial position is monitored by signalling means within the fixing means and transmitted to the cell phone or smartphone where it is processed by using a corresponding app. This app may be used for other purposes as well, i. e. to provide an operating manual for the device to help in error analysis etc.. It is further possible to activate an additional alert signal (snooze function) when a predetermined time has lapsed. The app may be operable to inform persons that support is needed. The app may activate further apps on the smartphone. The alert signal may be produced by the smartphone, i. e. by a vibrational alert, i. e. without disturbing other persons. It is possible to produce a distance signal in order to prevent that the suction device is forgotten.

In addition or alternatively mechanical means may be provided within the fixing means which is activated by return of the indicator wall to its initial position. In this case, neither a battery or any other source of energy nor a wireless connection is required. In case where no electronic means are provided, disposal of the device is easier. In a construction where the indicator wall is coupled mechanically to fixing means acoustic or haptic signals may be generated.

In a further embodiment of the invention acoustic signalling means optionally coupled to optic signalling means, like an alert LED, may be provided within fixing means and automatically activated in case the indicator wall returns into its initial position.

According to a further concept, a timer may be provided which is activated as soon as operation of suction means are stopped and activates an alert signal as soon as a predetermined time has lapsed. Corresponding timer means may also be associated with the suction means.

In addition or alternatively to the indicator devices explained above, a wound care system according to the invention can also have a tube connected fluidically to the wound space, which tube has a reduced pressure-stable section facing the wound face and a compressible section facing away from the wound space, the compressible section being closed fluid-tightly on its end facing away from the wound space, the tube being filled partially with a liquid, in particular white oil. The compressible tube section collapses due to reduced pressure generated by applying reduced pressure to the wound space and also in the tube connected fluidically to the wound space. The liquid is pushed out of this region and flows optionally to a mark on the reduced pressure-stable tube section which may correspond to the desired reduced pressure level. In this way a reduced pressure status can be displayed qualitatively.

In order to avoid influencing this indicator device by mechanical forces acting from the outside, the entire tube system can be encapsulated, for example by a protective tube. The display of the desired reduced pressure is set by the inside diameter of the tube, the flexibility of the tube material and the density of the fluid.

Overall, a wound care system according to the invention with an indicator device enables rapid and discrete monitoring of the reduced pressure status. If a rise in pressure is determined here, the latter can be subsequently corrected at any time by coupling the suction device. The monitoring of the reduced pressure and optionally the re-pumping can be implemented independently and intuitively by most patients and medical professionals, possibly after a short briefing.

A wound care system according to the invention may have a dressing already provided with a valve system and/or an indicator device. By structurally detaching a valve system from the reduced pressure source and positioning it in the dressing or in a valve system connected to the dressing by means of a tube, a reduced pressure pump connected directly to the wound care system may be dispensed with. The indicator device of a wound care system according to the invention may additionally or alternatively, acoustically, mechanically or visually display a rise in pressure, in particular according to a colour change. Furthermore, the use of electromechanical sensors is conceivable, with which sensors a signal representing the pressure and/or the rise in pressure is issued, possibly wirelessly, for example to a smartphone or the like.

As can be gathered from the above explanation of wound care systems according to the invention, it has proven to be particularly advantageous if this wound care system also has a suction device that can be coupled detachably to the valve system in addition to the covering device, the valve system and/or the indicator device.

This type of suction device can be realised, for example, in the form of a simple piston system which can preferably have a receiver for the valve body of the valve system assigned to the covering device. The suction device may have a sealing flange passing around the one-way valve of the valve body, by means of which a connection of the valve chamber to at least one suction chamber of the suction device can be established which on the one hand enables gases to pass out of the valve chamber into the suction chamber, but on the other hand counters the penetration of gases from the suction chamber into the valve chamber. For this purpose, after inserting the valve body into the receiver, the sealing flange may rest, forming a seal, against a limiting element of the valve chamber that comprises the one-way valve and pass around the one-way valve.

As already explained above in connection with the valve system, the system of the invention has proven to be particularly advantageous since the two limiting elements of the valve chamber are lens-shaped and are provided with a one-way valve. Thus the function of the suction device is not dependent upon the alignment of the valve body in the receiver of the suction device.

In one particularly preferred embodiment of the invention the receiver of the suction device designed to accommodate the valve body is formed between a housing that has the sealing flange and a counter-holder that can be adjusted relative to the housing between an open position and a closed position. If the counter-holder is placed in the open position, the valve body can be introduced into the receiver. By closing the counter-holder the suction process can be initiated.

Advantageously, the suction device can be operated to compress the valve chamber of the valve body held in the receiver such that gases are pushed and/or sucked out of the valve chamber by the one-way valve integrated into a limiting element thereof. The valve body is fixed with the counter-holder so that the sealing flange passes around the one-way valve, forming a seal. In this way the gas can pass from the valve chamber, through the one-way valve, into the suction chamber of the suction device which is connected, forming a seal, to the valve chamber with the aid of the sealing flange.

In one particularly preferred embodiment of the invention the suction device has at least one suction piston that can advantageously be introduced into the suction chamber against the pre-tensioning force of a pre-tensioning device. In this way the available volume of the suction chamber is reduced. In one preferred embodiment of the invention the pre-tensioning force can be provided by a compression spring passing around helically and positioned between a bottom of the suction chamber and the suction piston.

By reducing the available volume of the suction chamber the gas accommodated within it would be compressed. However, a further valve system is assigned to the suction piston, which valve system allows gases to be released from the suction chamber to the surrounding area when the suction piston is introduced into the suction chamber so as to avoid excessive compression of the gases accommodated in the suction chamber and countering the penetration of ambient air into the suction chamber when the suction piston is removed from the suction chamber. In this way the gases accommodated in the suction chamber are not compressed in the suction chamber when the suction piston is introduced, but are released into the surrounding area by the additional valve system. Suction is generated by increasing the volume of the suction chamber by removing the suction piston from the latter. By means of this suction gases are conveyed out of the valve body, and so out of the wound space connected to the valve body through the one-way valve provided in a limiting element of the valve body and the sealing flange of the suction device into the suction chamber, while at the same time the suction chamber is sealed with respect to the surrounding area by the additional sealing system assigned to the suction piston. Thus, with the aid of the suction device and the valve body held in its receiver, reduced pressure can be generated in the wound space.

As explained earlier, in a particularly preferred embodiment of the invention, the volume wherein a reduced pressure is increased by providing an additional valve chamber and/or indicator chamber. A further increase of the volume wherein reduced pressure is generated which helps to maintain pressure below a pre-determined threshold over an extended period of time is provided by a wound care system comprising a valve chamber, preferably having said indicator wall, and an indicator chamber in fluid communication with said valve chamber wherein said indicator chamber is at least partially delimited be a deformable wall which is urged into the indicator chamber by applying a reduced pressure to the valve chamber and thus, due to the fluid communication, also to the indicator chamber. In this embodiment of the invention, the indicator chamber has a double function of indicating the pressure status within the wound space on the one hand and on the other hand providing an additional volume wherein reduced pressure is generated. In case the deformable wall is urged into the indicator chamber due to the pressure difference between the interior of the indicator chamber and ambient atmosphere, haptic control of the pressure status is possible.

In addition to haptic control or as an alternative to haptic control, optical control of the pressure status is possible, In case the valve chamber is separated from the indicator chamber by a separating wall penetrated by a fluid communication hole wherein said separating wall extends essentially parallel to said deformable wall, when reduced pressure is produced within the indicator chamber, the deformable wall is urged against the separating wall. In case some type of logo or written indication is printed on the surface of the separating wall facing the deformable wall, such indication becomes visible in case the deformable wall touches the separating wall.

In a further preferred embodiment of the invention said valve chamber and said indicator chamber are at least partially delimited by a rigid housing, preferably by a rigid circumferential chamber jacket which is at least partially accommodated in an outer jacket which at least partially is formed by a soft material also forming the deformable wall. In this embodiment of the invention the deformable wall may be formed integrally with the outer jacket. The soft outer jacket may help to improve wearing comfort of the device and may ensure adequate attachment of the deformable wall.

In a further preferred embodiment of the invention, a cylinder-piston arrangement or sleeve-in-sleeve arrangement may be accommodated within the valve chamber, wherein a cylinder or piston is sealingly attached to the indicator wall and sealingly connected to another piston or cylinder member such that respective members may be shifted with respect to each other. The overall arrangement may accommodate a pressure spring which urges respective members apart from each other. In case reduced pressure is applied to the valve chamber and thus also to the interior of the cylinder-piston assembly, ambient pressure urges the cylinder-piston assembly into a compressed state. In this state the bottom wall of the cylinder-piston arrangement facing away from the indicator wall may be retracted from a transparent wall member of an outer housing to thereby enable optical control of the reduced pressure status. In case the bottom wall of the cylinder-piston arrangement touches the transparent wall, undue increase of the pressure within the valve chamber and thus also within the wound space may be detected. In case reduced pressure is generated within the wound space and thus also within the valve chamber and the interior of the cylinder-piston assembly, the bottom wall is retracted from the transparent wall against the pre-tension force of the pressure spring accommodated within the cylinder-piston assembly. This may be noted visually to thereby provide visual control of the reduced pressure status.

As can be gathered from the above explanation of wound care systems according to the invention, a kit suitable for producing this type of wound care system comprises a preferably sterilely packaged covering device, a valve system, an indicator device and/or a suction device which can be coupled to the valve system. This type of kit can be assembled so that it only has a covering device, a valve system and/or an indicator device, whereas the suction device can be used for a number of these types of kit. However, the kit may also comprise the suction device as an independent element. Here, the suction device may also be made in the form of a pump, in particular a mechanical or an electromechanical pump.

An indicator device according to the invention suitable for producing a wound care system according to the invention has, as can be gathered from the above explanation of the invention, a chamber which can be connected fluidically to a wound space and is delimited by a circumferential chamber jacket, the chamber preferably having an indicator wall that can be moved between an initial position and a final position, and that in particular has a snap disc, and the indicator wall can be moved from an initial position into the final position by applying a reduced pressure to the chamber, and upon pressure equalisation passes automatically into the initial position. The indi-cator device may have an at least partially transparent monitoring wall which is preferably deformable and, upon reaching a pre-defined reduced pressure in the chamber, i.e. after the indicator wall has passed into the final position, comes into contact with the indicator wall.

The invention is explained below with reference to the drawings to which reference is explicitly made with regard to all of the details which are essential to the invention and are not elaborated any further in the description. The drawings show as follows:
- Fig. 1: a wound care system after application to a wound,
- Fig. 2: a valve system of a wound care system according to the invention according to a first embodiment of the invention,
- Fig. 3: a valve system of a wound care system according to the invention according to a second embodiment of the invention,
- Fig. 4: a valve system of a wound care system according to the invention according to a third embodiment of the invention,
- Fig. 5: an indicator device of a wound care system according to the invention according to a fourth embodiment of the invention,
- Fig. 6: an indicator device of a wound care system according to the invention according to a fifth embodiment of the invention,
- Fig. 7: a suction device of a wound care system according to the invention in a first operating position,
- Fig. 8: the suction device according to Fig. 4 in a second operating position,
- Fig. 9: a combined valve and indicator device of a wound care system according to the invention according to a sixth embodiment of the invention, and
- Fig. 10: a schematic illustration intended to explain the function of the sixth embodiment of the invention,
- Fig. 11: a sectional illustration of a seventh embodiment of the invention,
- Fig. 12: a schemantic illustration of an eight embodiment of the invention,
- Fig. 13: a schematic illustration of a ninth embodiment of the invention,
- Fig. 14: A schematic illustration of a tenth embodiment of the invention, and
- Fig.15: a suction device that can be used in association with the embodiment of the invention illustrated in Fig. 12 and 13.

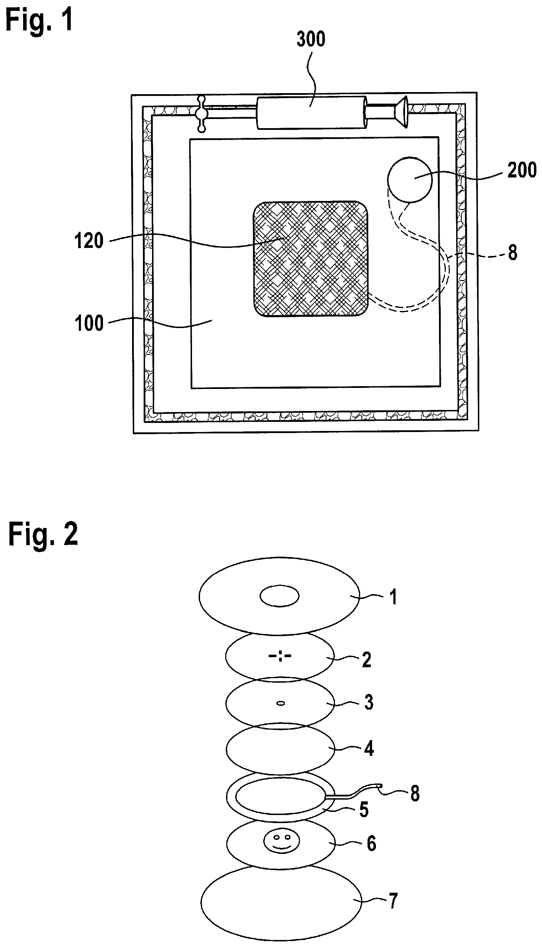

The wound care system illustrated in Fig. 1 essentially comprises a covering device 100 realised in the form of a transparent, gas-tight and preferably water vapour permeable polyurethane film, a wound filling material 120 and a valve system identified as a whole by 200 which is connected to the wound space by means of a tube 8. The polyurethane film 100 is provided on its side facing the skin with an adhesive agent. The tube 8 runs between the covering device 100 and the skin and leads into the wound space configured with the wound filling material 120. Assigned to the valve system 200 is a suction device 300 that can be detachably coupled to the latter in the form of a piston pump which, in the operating position illustrated in Fig. 1, is detached from the valve system 200. In the wound space configured with the wound filling material 120, reduced pressure can be generated by the tube 8 and the valve system 200 with the aid of the suction device 300.

In Fig. 2 a valve system with an integrated indicator device of a wound care system according to a first embodiment of the invention is illustrated in exploded form. The valve system comprises two limiting elements 2 and 7 which are connected to one another in the region of their edges, optionally indirectly, forming a seal, so as to form a valve chamber. The valve chamber that is formed in this way is in the form of a lens and serves to receive gases from the wound space via the tube 8. In the limiting element 2 valve slots are provided to form a one-way valve or check valve. The one-way valve makes it possible to discharge gases from the valve chamber and counters the conveyance of gases from the surrounding area into the valve chamber. Accordingly, gases can only be conveyed into the valve chamber via the tube 8, and so out of the wound space. The limiting element 2 is covered with a reinforcement strip 1, the reinforcement strip 1 having a recess in order to keep the valve slots free. Disposed between the limiting elements 2 and 7 is a film 3 additionally provided with a hole. An indicator device is held between the film 3 and the limiting element 7. The indicator device comprises two indicator layers 4 and 6 running spaced apart and parallel to one another as well as a spacer layer 5 made of compressible material disposed between the indicator layers 4 and 6. The spacer layer 5 is made to be annular in form and the tube 8 passes through it. An indicator space is formed between the indicator layers 4 and 6 and the spacer layer 5.

Due to a pressure difference between the pressure in the indicator space, which because of the connection via the tube 8 corresponds to the pressure in the wound space, and the ambient pressure, the indicator layer 4 is pushed against the pre-tensioning force brought about by the spacer layer 5 against the indicator layer 6. The spacer layer 5 bringing about the pre-tensioning force can have different types of elastic elements with a defined restoring force, such as for example springs, e.g. helical or disc springs. However, foam materials or spacer fabrics may also be used which can be compressed against a pre-defined pre-tensioning force. On the boundary surface of the indicator layer 6 facing the indicator layer 4 an image motif is provided which is visible when the indicator layer 4 comes into contact with the indicator layer 6. It can thus be monitored whether the reduced pressure in the wound space, which corresponds to the pressure in the indicator space, still meets the requirements.

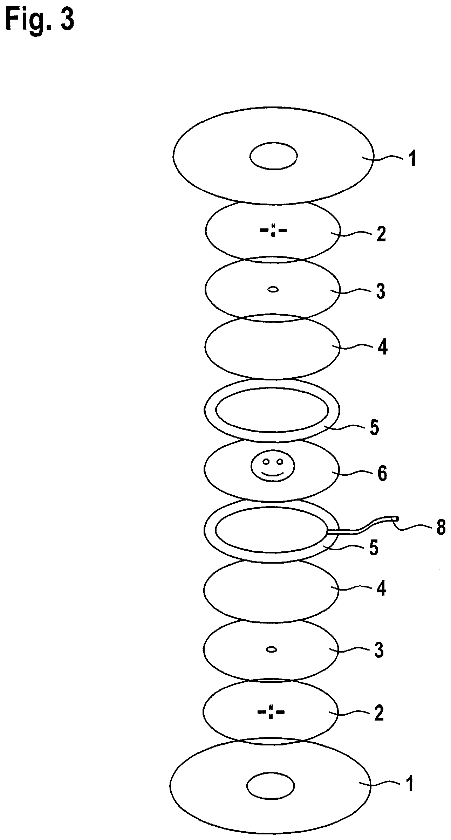

The embodiment of the invention illustrated in Fig. 3 differs substantially from the embodiment of the invention explained by means of Fig. 2 in that an identical structure with a spacer layer 5, an indicator layer 4, a film with an air hole 3 and a limiting element 2 made with valve slots is provided on both sides of the indicator layer 6, in the region of both limiting elements reinforcement layers 1 being provided which have a recess enabling air to escape from the valve chamber formed between the limiting elements 2.

The valve systems of the embodiments of the invention illustrated in Figures 2 and 3 comprise film valves in the sense explained above in the region of the limiting elements 2 and 7. In the embodiments of the invention illustrated in Figures 2 and 3 a combination of a film valve and an indicator device is respectively illustrated. In the embodiment illustrated in Fig. 3, there is a film valve on both sides. The valve chamber can then be evacuated from both sides.

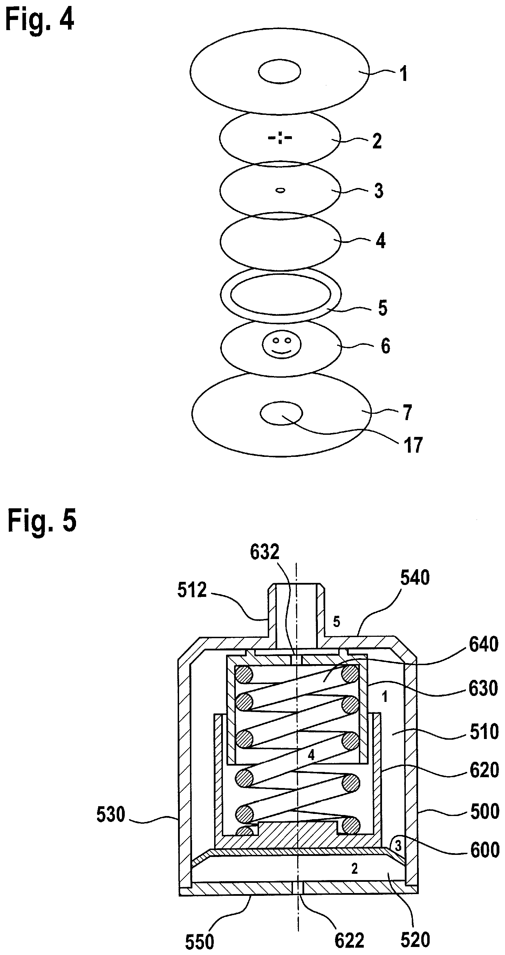

The valve system of a wound care system according to the invention illustrated in Fig. 4 corresponds substantially to the valve system explained by means of Fig. 1. However, the valve system illustrated in Fig. 4 is designed to be applied directly to the covering device of the wound care system. It does not have to be connected to the wound space by means of a fluid line. In the embodiment of the invention illustrated in Fig. 4 the fluid conveying connection to the wound space can be established by means of a hole 17 in the limiting element 7, this limiting element 7 being made in the form of a self-adhesive covering film. This self-adhesive covering film 7' with the centrally positioned hole 17 can be located over a perforation in the covering device (not illustrated). The wound space can be evacuated directly by means of the film valve and the hole 17 in the self-adhesive covering film and the perforation in the covering device. Moreover, the function of the valve system according to Fig. 4 corresponds to the function of the valve system explained by means of Fig. 2.

In Fig. 5 an indicator device that can be used within the framework of the invention and that has a rigid housing identified as a whole by 500 is illustrated. The rigid housing 500 is made overall in the form of a circular cylindrical jacket and has two substantially level front surfaces 540 and 550 as well as a jacket 530. The front walls 540, 550 and the jacket 530 of the housing are produced from a transparent material, in particular from a transparent plastic. In the housing 500 two chambers 510 and 520 separated from one another in a gas-tight manner by a deformable membrane 600 are formed. The chamber 520 is connected to the surrounding area by a hole 622 in the front wall 550 of the housing 500. The chamber 510 can be connected fluidically to a wound space by means of a tube port 512 which is disposed in the region of the front surface 540. Two sleeves 620 and 630 which engage in one another and can be displaced with respect to one another are accommodated within the chamber 510. The sleeve 620 is coupled to the membrane 600, whereas the sleeve 630 is coupled to the front wall 540. The sleeves are pushed apart from one another with the aid of a pre-tensioning device 640 made in the form of a compression spring. The jacket of the sleeve 620 coupled to the membrane 600 has a larger diameter than the jacket of the sleeve 630 coupled to the front surface 540, which sleeve 630 is connected to the chamber 610 by means of a hole 632 formed on a face surface thereof. The membrane 600 lies against the front wall 550 of the chamber 520 without generating reduced pressure in the chamber 510. In this initial position the jacket of the sleeve 630 is only covered by the sleeve 620 within the framework of an edge opposite the front wall that has the hole.

If the sleeves 620 and 630 are coloured differently, for example by the sleeve 630 being coloured red and the sleeve 620 being coloured green, it can be seen through the transparent jacket 530 of the housing 500 that there is insufficient reduced pressure in the chamber 510 and so also in the wound space. If reduced pressure is generated in the chamber 510, for example by means of the suction port 512, the membrane 600 may be lifted from the front wall 550 of the chamber 520 due to the corresponding suction effect, pressure equalisation being able to take place in the chamber 520 by means of the opening 622 in this front wall. By lifting the membrane 600 from the front wall 550, the sleeve 620 accommodated in the chamber is pushed towards the front wall 540 of the housing 500 against the pre-tensioning force of the compression spring 640 and so overlaps the sleeve 630. By means of the different colourings of the sleeves 620 and 630, it can thus be signalled through the transparent housing 500 that there is sufficient reduced pressure in the chamber 510, and so also in the wound space.

When the reduced pressure drops, the compression spring 640 pushes the sleeve 620 back in the direction of the front wall 550. The jacket of the sleeve 630 can be seen once again. It is thus signalled through the transparent jacket 530 of the housing 500 that action is necessary in order to obtain the desired reduced pressure status. The concept explained by means of Fig. 5 is also possible in other variations. For example, the two sleeves 620 and 630 can swap positions. For example, the seal between the chambers 510 and 520 can be configured as a flexible setback lip seal.

By applying reduced pressure to the chamber 520 with the aid of an appropriate suction device, air flows through the lip seal from the direction of the chamber 510 and the dressing connected thereto (not illustrated). After completion of the active evacuation the position of the piston (with seal) shifts, according to the equilibrium, from the force resulting from the reduced pressure and the force of the compression spring countering the latter. As a result, the positional relationship of the sleeves 620 and 630 also shifts. The system can be set up so that with the desired reduced pressure status, the sleeve 630 is completely covered by the sleeve 620, and so is no longer visible.

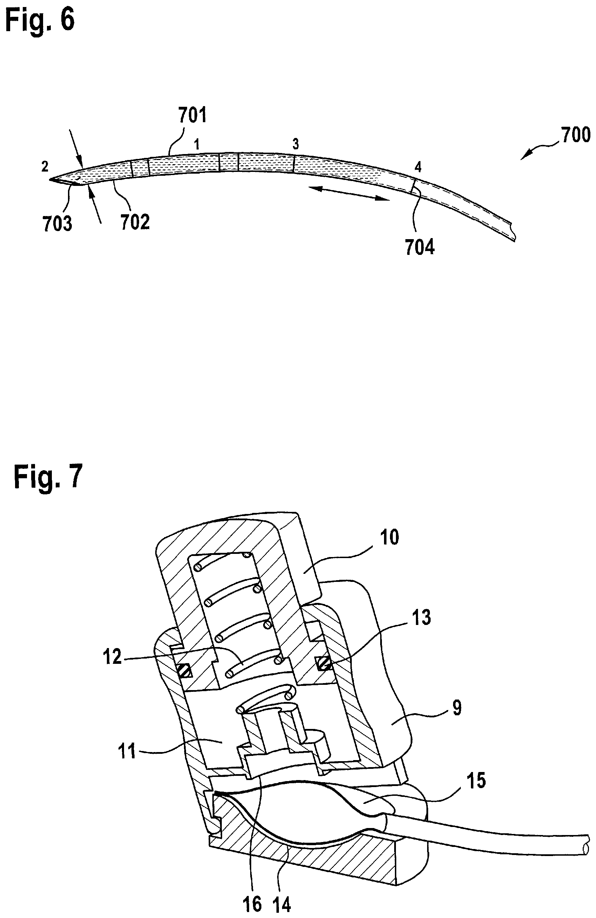

In the indicator device illustrated in Fig. 6, a tube identified as a whole by 700 is used for the visual representation of the reduced pressure status. The tube 700 comprises a reduced pressure-stable tube section 701 and a compressible tube section 702. The two tube sections 701 and 702 are connected to one another in an air- and liquid-tight manner. The tube section 702 is closed, airtight, on its end facing away from the tube section 701. In this way a type of closed blister is produced. The end of the tube section 701 facing away from the tube section 702 is connected fluidically to the wound space. A check valve can also be disposed here between the wound space and the tube section 702.

The tip of the tube 700 which is produced by closing the end of the tube section 702 facing away from the tube section 701 is filled up to a pre-specified mark with a liquid 703. Medical white oil, for example, can be used here. Preferably, the tube as a whole is produced from a polymer which only has a small degree of wettability due to the oil so as to enable a rapid outflow of the liquid if there is a decrease in reduced pressure. The tube section 702 collapses due to the reduced pressure that is generated upon evacuating the wound space. The liquid 703 is pushed out of this region and flows to a second mark 704 on the pressure stable tube section 701. The position of this mark corresponds to the desired reduced pressure level. The reduced pressure status can thus be qualitatively displayed and monitored. In order to avoid any negative impact upon the functional capability of the indicator device illustrated in Fig. 5, for example due to mechanical forces, the entire tube can be encapsulated, for example, by a protective tube. The display of the desired reduced pressure is set by the inside diameter of the tube, the flexibility of the tube material and by the density of the liquid.

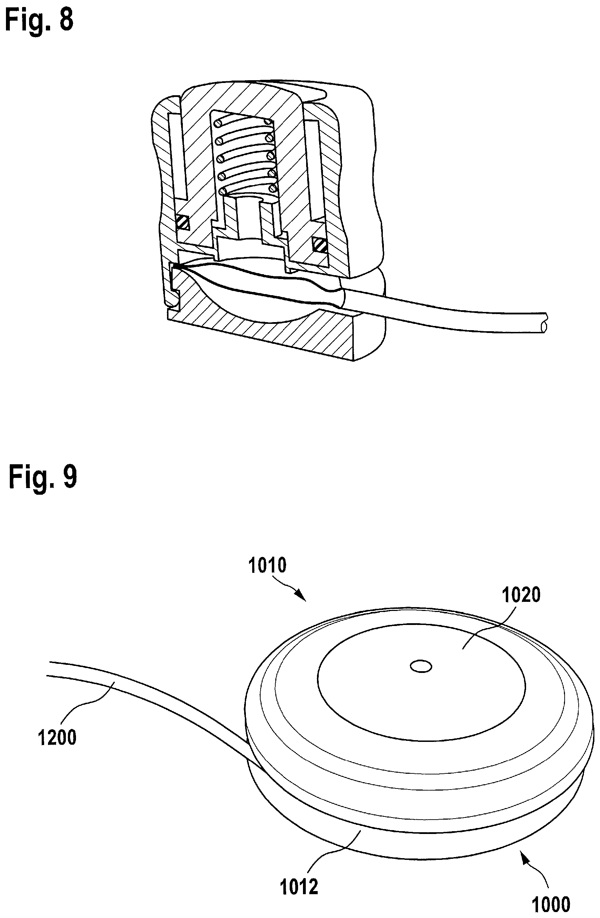

The suction device illustrated in Figures 7 and 8 essentially comprises a housing 9 provided with a sealing flange 16, a piston 10 that can be introduced into the housing against the pre-tensioning force of a spring 12 and a counter-holder 14 that can be moved relative to the housing 9 between an open position, which is illustrated in Fig. 7, and a closed position, which is illustrated in Fig.8. In the open position of the counter-holder 14 illustrated in Fig. 7 a valve body 15 of the type illustrated in Figs. 2 and 3 is introduced into a receiver formed between the housing 9 and the counter-holder 14. A limiting element, provided with valve slots, of the valve body held in the receiver lies against a sealing flange of the housing 9 such that gases can pass out of the valve chamber into the suction chamber formed by the housing 9. By adjusting the counter-holder relative to the housing 9 from the open position illustrated in Fig. 7 into the closed position illustrated in Fig. 8, the valve body 15 is fixed. The fixing of the valve body is brought about so that the sealing flange passes around the one-way valve of the valve body, forming a seal. By actuating the suction device the gases can be sucked out of the valve body and the wound space through the valve slots. In this way the valve body, and so also the wound space, are evacuated. If the suction piston 10 is introduced into the suction chamber 11 against the pre-tensioning force of the compression spring 12, a valve ring lying on a flange of the suction piston is lifted from the flange and makes it possible for gases to escape from the suction chamber 11 into the surrounding area.

If the suction piston 10 is pushed out of the suction chamber 11 by the pre-tensioning force of the spring 12, the valve ring 13 lies against the flange once again and seals off the suction chamber from the surrounding area. The suction that is produced by pushing the suction piston out of the suction chamber by expanding the volume of the suction chamber causes gases from the valve chamber of the valve body 15 to be introduced and so the generation of a reduced pressure in the wound space connected to the valve body 15 by the tube 8. As soon as sufficient reduced pressure has been generated in the wound space, when the counter-holder 14 is open the valve body 15 can be removed from the receiver and the suction device as a whole is mounted independently of the valve system connected to the covering device by the tube 8 and transported. The valve system or the valve body 15 can then be locked in the region of the covering device close to the wound. As soon as a rise in pressure is determined in the valve body 15 or in the wound space with the aid of the indicator device which may be integrated into the valve system, the valve body 15 can once again be coupled to the suction device and gases can once again be sucked out of the wound space.

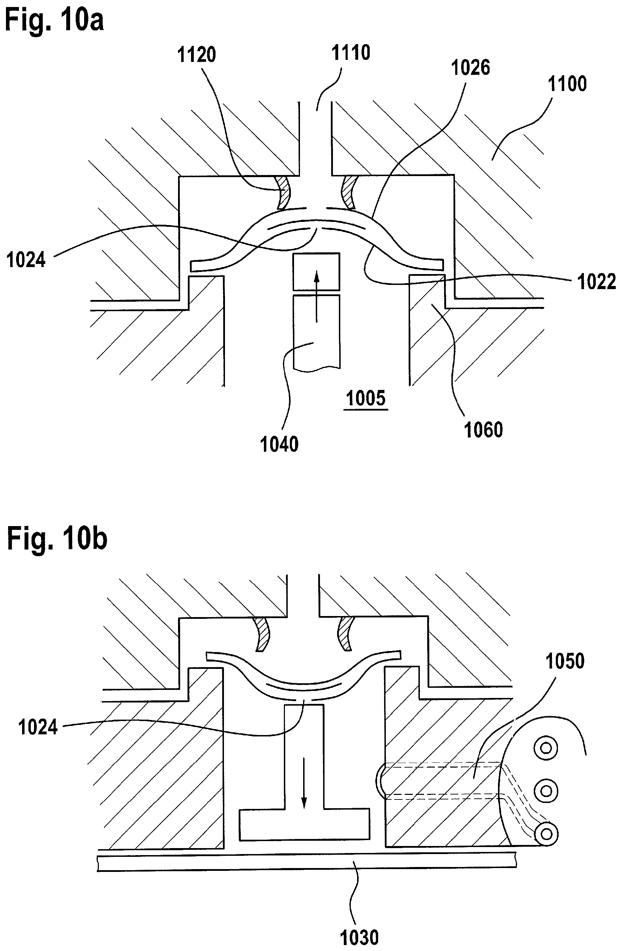

The embodiment of the invention illustrated in Fig. 9 has a combined valve and indicator system identified as a whole by 1000. A chamber 1005 around which a chamber jacket 1010 passes (see Fig. 10) is provided here. The chamber 1005 and the chamber jacket 1010 are made as a whole to be approximately rotationally symmetrical, the height of the substantially cylindrical chamber 1005 being substantially less in the direction of the rotational axis of the chamber jacket 1010 than the diameter of the chamber jacket 1010. A front surface of the chamber 1005 is formed by an indicator wall identified overall by 1020. As can be gathered from the illustration in Fig. 10a and 10b, the indicator wall comprises a snap disc 1022 which curves convexly outwards in the force-free state and through which a recess 1024 passes in the crest region of the curve, and which is fixed to the inner edges of the chamber jacket 1010 adjacent to the chamber 1005.

On its side facing away from the chamber 1005 the recess 1024 is covered by a film valve identified as a whole by 1026. The film valve 1026 makes it possible to suck fluids out of the chamber 1005, but counters the penetration of fluids into the chamber 1005. In this way the chamber 1005 can be evacuated with the aid of a schematically indicated suction device identified as a whole by 1100. On a boundary surface facing the indicator wall in the operating position the suction device 1100 has a seal system 1120 passing around a suction channel 1110 which can be applied to the indicator wall 1020 such that a gas-tight connection between the suction channel 1110 and the indicator wall 1020 is established.

The seal system 1120 passes around a schematically indicated valve slot of the film valve 1026 by means of which a fluid can be sucked out of the chamber 1005 via the suction channel 1110. As soon as reduced pressure is generated in the chamber 1005, there is a pressure difference between the pressure in the chamber 1005 and the pressure outside of the seal system 1120 outside of the seal system 1120. In this way a force pushing the indicator wall 1020 into the chamber 1005 is generated. As soon as the pressure difference is sufficiently great, the snap disc 2011 together with the film valve 1026 disposed on top of it folds down from the initial position illustrated in Fig. 10a into the final position illustrated in Fig. 10b in which the snap disc 1022 is curved into the chamber 1005. The sealing coupling of the suction channel to the indicator wall is released.

The automatic release of the suction channel from the indicator wall is assisted by the chamber jacket having a circumferential collar 1060 made in the form of an annular protrusion which is held in a recess of the suction head of the suction device 1100 so that a front side boundary surface of the suction device 1100 comes into contact with a boundary surface of the chamber jacket 1010 passing around the annular protrusion. The chamber jacket then forms a stop limiting the approach of the seal system 1120 to the snap disc 1022 or the recess 1024 passing through the snap disc so that the seal system 1120 cannot follow the snap disc 1022 into the end position when folded over from the initial position.

It can be determined haptically whether the indicator wall 1020 is curved outwardly (Fig. 10a) or inwardly (Fig. 10b). Thus, the reduced pressure status in the chamber and so also in the wound space connected to the chamber 1005 (see below) can be checked. The snap disc 1022 is made here overall such that in the force-free state, i.e. when the same pressure conditions prevail inside and outside of the chamber 1005, it automatically adopts the initial position illustrated in Fig. 10a. On the side facing away from the indicator wall the chamber 1005 is delimited by an at least partially transparent monitoring wall 1030 on the outer boundary surface of which an adhesive agent, such as for example a hooked strip of a surface hook and loop fastener, can be provided.

Provided within the chamber 1005 is an additional indicator element 1040 which is coupled to the snap disc 1022. If upon reaching a desired reduced pressure status in the chamber 1005 the snap disc 1022 is folded down from the initial position illustrated in Fig. 10a into the final position illustrated in Fig. 10b, a boundary surface of the indicator element 1040 comes into contact with the monitoring wall 1030. As a result, visual monitoring of the reduced pressure status is also made possible in addition to haptic monitoring.

As can be seen in Fig. 10b, a channel 1050 extending in the radial direction passes through the chamber jacket 1010. On its side facing away from the chamber 1005 the channel 1050 leads into a circumferential outer groove 1012 in an outer boundary surface of the chamber jacket 1010. The channel 1050 serves to establish a fluidic connection between the chamber 1005 and the wound space. For this purpose a tube 1200 can be provided which leads into the channel 1050 or is accommodated in the channel 1050 and leads directly into the chamber 1005. The tube 1200 can be wound up in the circumferential groove formed in the chamber jacket. The tube length can thus be set according to the requirements.

The walls of the groove 1012 can be provided with indentations in the region of their ends facing away from the groove bottom. In the region of the indentations the groove width may correspond approximately to the diameter of the tube. Outside of the indentation the groove width may be smaller than the diameter of the tube. Locking of the tube emerging out of the tube at a desired length can thus be brought about. It is then only possible to change the length by slightly squeezing the tube and/or deforming the groove walls, i.e. with additional expenditure of force.

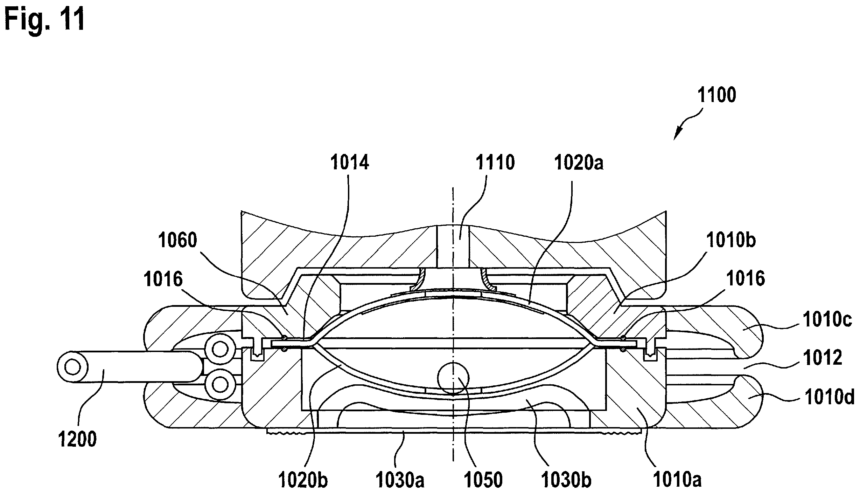

The embodiment of the invention illustrated in Fig. 11 essentially differs from the embodiment explained by means of Fig. 10 in that the chamber jacket 1010 is made in a number of parts. Parts 1010a and 1010b of the chamber jacket 1010 of the embodiment of the invention illustrated in Fig. 11, which lie radially on the inside, are made of a comparably hard plastic material, an inner groove 1014 being formed between the bottom part 1010a and the top part 1010b in which a circumferential edge region of the indicator wall 1020 is accommodated. The indicator wall made in the form of a snap disc is illustrated in the initial position by 1020a and in the final position by 1020b. Sealing rings 1016 are inserted in the region of the walls of the inner groove 1014. In this way the circumferential edge region of the indicator wall 1020 is held, forming a seal, in the inner groove 1014. A space is left free between the bottom of the inner groove 1014 and the edge region of the indicator wall 1020 in the initial position 1020a and the final position 1020b. In this way radial deformation of the indicator wall 1020 when folding down from the initial position into the final position is made possible. The radially outer parts 1010c and 1010d of the chamber jacket 1010 are made of a comparably soft plastic material, and they can be deformed to move the tube 1200 out of the outer groove 1012 formed between the chamber jacket part 1010c and the chamber jacket part 1010d. The axially inwardly pointing edges of the chamber jacket parts 1010c and 1010d may have indentations in the region of which the space between the edges of the chamber jacket parts 1010c and 1010d facing one another corresponds approximately to the tube diameter. In this way a lock can be formed for the tube 1200 emerging out of the outer groove 1012.

In the embodiment of the invention illustrated in Fig. 11, the monitoring wall 1030 is formed from a deformable material. In the final position of the monitoring wall illustrated in Fig. 11 by 1030b, the monitoring wall nestles against the indicator wall 1020b which in this state is in the final position, aided by the pressure difference between the chamber interior and the chamber exterior. In this way visual monitoring of the reduced pressure status in the chamber, and so also in the wound space, can take place. For this purpose, the boundary surface of the indicator wall 1020 facing the monitoring wall 1030 can be provided with profiling or a graphical representation.

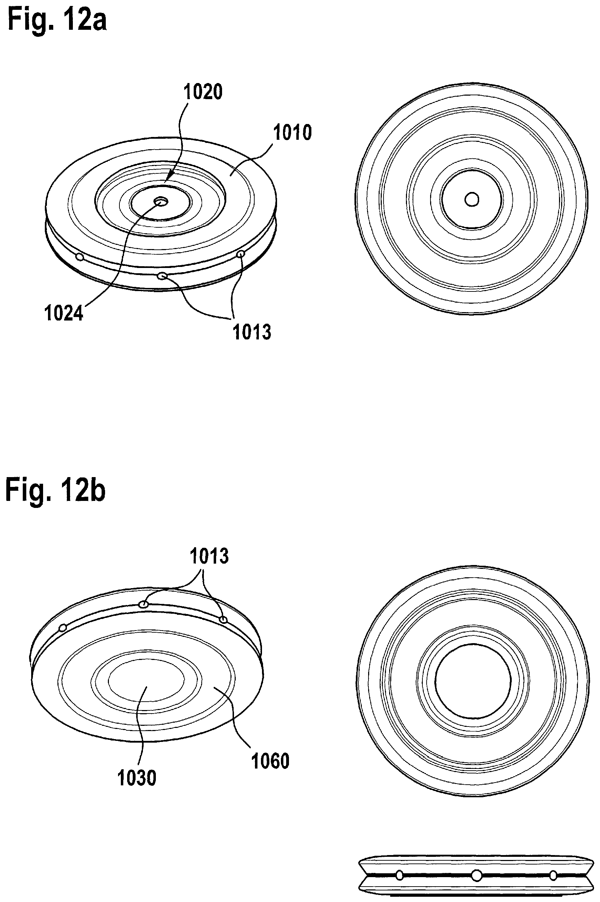

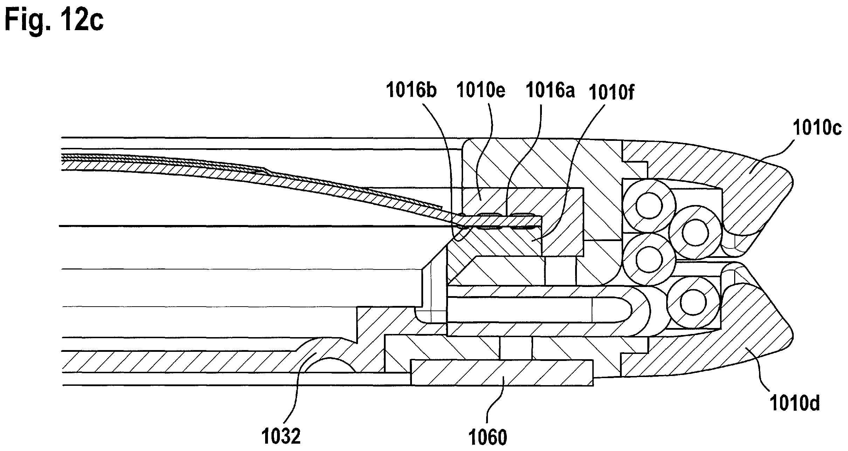

Fig. 12a shows a perspective illustration obliquely from above of an indicator device according to another embodiment of the invention. Fig. 12b shows a perspective illustration obliquely from below of the indicator device according to Fig. 12a, and Fig. 12c shows a sectional illustration of the indicator device according to Figs. 12a and 12b. The embodiment shown in Fig. 12 substantially corresponds in terms of its function to the embodiment illustrated by Fig. 11. It essentially differs from the embodiment illustrated by Fig. 11 in that the cross-over between the indicator wall 1020 and the chamber jacket 1010 is made in the form of a setback so that a receiver for a suction head of a suction device is formed between the chamber jacket 1010 and the indicator wall 1020, the edge of the chamber jacket 1010 lying radially on the inside forming a stop limiting the approach of the suction head to the indicator wall 1020, and so also the recess 1024 passing through the indicator wall.

In Figs. 12b and 12a the indentations in the chamber wall parts 1010c and 1010d, which form a lock for the tube emerging out of the outer groove 1012, can be seen particularly clearly at 1013. As can be seen particularly clearly in Fig. 12b, in the embodiment of the invention illustrated in Fig. 12 a ring 1060 made of adhesive material, preferably in the form of a hooked strip of a surface hook and loop fastener, will pass around the monitoring wall 1030. In this way the attachment of the indicator device to the covering device of a wound care system according to the invention is assisted.

As can be seen in Fig. 12c, the monitoring wall 1030 has a ring 1032 that is curved towards the indicator wall 1020. Due to this annular structure 1032 a sufficiently large amount of material is provided to enable deformation of the monitoring wall 1030, as is required to place the monitoring wall 1030 against the indicator wall in the final position without expanding the material of the monitoring wall. Moreover, the embodiment according to Fig. 12 also differs from the embodiment according to Fig. 11 in that the receiver, forming a seal, of a circumferential edge region of the indicator wall 1020 in the inner groove 1014 is assisted by chamber wall parts 1010e and 1010f made of a flexible material, the chamber wall parts 1010e and 1010f still also having circumferential sealing ribs 1016a projecting into the groove interior.

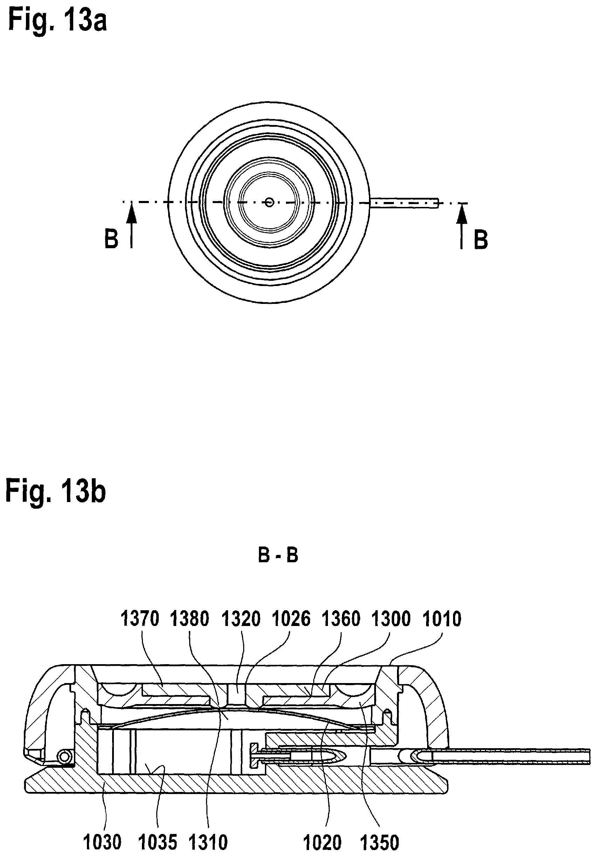

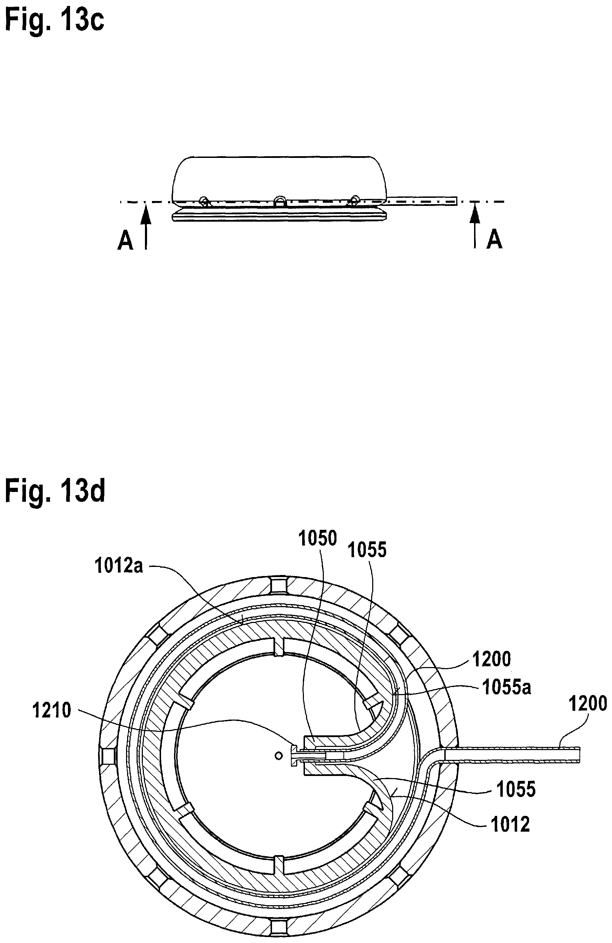

Fig. 13a) illustrates a top view of the ninth embodiment of the invention. Fig. 13b) illustrates a cross-sectional view of the ninth embodiment taken along cross-sectional plane BB shown in Fig. 13a). Fig. 13c) shows a side-elevational view of the ninth embodiment. Fig. 13d) shows a cross-sectional view taken along cross-sectional plane A-A as shown in Fig. 13c).

The embodiment shown in Fig. 13 mainly differs from the embodiments shown in figures 11 and 12 by providing a rigid support member 1300 for the film valve 1026. Thus, the rigid support member 1300 forms part of the end wall of the valve chamber and the indicator wall 1020 which is realized by means of a snap disc is accommodated within the valve chamber. The indicator wall is curved convexly outwardly in its initial position into a room defined between the indicator wall 1020 and the rigid support member 1300.

In this initial position an abutting surface 1310 of rigid support member 1300 abuts against indicator wall 1020. Film valve 1026 is attached to an outer surface of rigid support member 1300 facing away from abutting surface 1310. Rigid support member 1300 is attached to the circumferential chamber jacket 1010 by means of an elastically deformable coupling member 1350 which allows rigid support member 1300 to be shifted into the valve chamber and to thereby urge the snap disc 1020 downwardly until it snaps into its final position (not shown).

The rigid support member 1300 in its central portion thereof comprises a recess 1320 in alignment with the central recess in snap disc 1020. In order to provide a form-fit engagement of rigid support member 1300 within coupling member 1350, coupling member 1350 is provided with a circumferential profile 1360 which accommodates a disc shape coupling portion 1370 of rigid support member 1300. Rigid support member 1300 is further provided with an annular protrusion 1380 running around recess 1320 and towards snap disc 1020, which annular protrusion 1380 on the one hand improves form-fit engagement of support member 1300 and coupling member 1350 and on the other hand provides an abutting surface 1310 via which rigid support member 1300 may abut against snap disc 1020.

In the embodiment shown in fig. 13, the monitoring wall 1030 is made of a rigid material. The bottom wall 1030 is provided with a recess 1035 to thereby increase the volume within the valve chamber which helps to preserve reduced pressure in the system over an increased period of time.

As can be seen in fig. 13d), outer groove 1012 of chamber jacket 1010 is connected to channel 1050 via curved transition areas 1055 which curved transition areas 1055 on the one hand merge tangentially into channel 1050 and on the other hand merge tangentially into a bottom wall of groove 1012. These curved transition areas 1055 provide guiding surfaces for the tube 1200 which help to avoid kinking or folding of the tube 1200 in the region between outer groove 1012 and channel 1050. For this purpose the radius of curvature of the guiding surfaces 1055a of the transition areas 1055 have a radius of curvature which is at least 10 %, preferably 20 5 or more of the radius of the bottom wall 1012a of outer groove 1012.

As can be seen in fig. 13d), a stop member 1210 is provided which is inserted in an end portion of the tube 1200 directed towards the valve chamber 1005. Stop member 1210 helps to prevent pulling out tube 1200 from channel 1050.

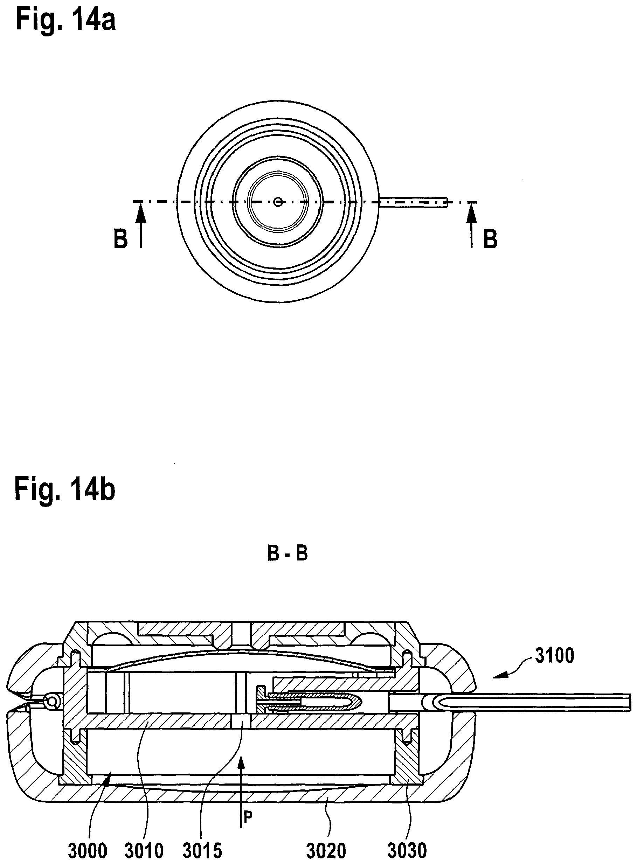

The embodiment shown in fig. 14 comprises a valve chamber accommodating a snap disc and being delimited by a film valve attached to a rigid support member. So far, the embodiment shown in fig. 14 corresponds to the embodiment shown in fig. 13. The main difference between the embodiment shown in fig. 13 and the embodiment shown in fig. 14 is the provision of an indicator chamber 3000 which is in fluid communication with a valve chamber via a through hole 3015 provided within a separating wall 3010 separating the valve chamber and the indicator chamber.

The indicator chamber is delimited by the separating wall 3010, a circumferential jacket member 3030 arranges coaxially to the circumferential chamber jacket of the valve chamber and a deformable wall 3020 running essentially perpendicularly to the separating wall 3010.

When applying a reduced pressure to the indicator chamber 3000 due to the pressure difference between the interior of the indicator chamber 3000 and ambient air, the deformable wall 3020 is urged into the interior of the indicator chamber as illustrated schematically by arrow P in fig. 14. By appropriately selecting rigidity of the deformable wall 3020 this deformable wall touches separating wall 3010 and thereby enables on the one hand haptic control of the reduced pressure status within the indicator chamber, valve chamber and the wound space, and on the other hand optical control of the reduced pressure status.

The deformable wall 3020 is preferably formed of a transparent material and particularly preferred formed integral with an outer jacket 3100 accommodating the indicator chamber 3000 and the valve chamber. Outer jacket 3100 on the one hand offers protection to the indicator chamber and the valve chamber and on the other hand improves wearing comfort of the overall device.

As an alternative to the embodiment shown in fig. 14, an arrangement of the type shown in fig. 5 may be attached to the snap disc in a fluid-tight manner such that the interior of the cylinder-piston arrangement or sleeve-in-sleeve arrangement of the type shown in fig. 5 is in fluid communication with the wound space. Thus, when reduced pressure is generated within the wound space, a bottom wall of the sleeve-in-sleeve or cylinder-piston arrangement against a pre-tensioning force of a pressure spring is retracted from deformable wall 3020 which in this case may also be replaced by a rigid transparent wall which is connected to ambient air via a through hole or the like. In case pressure increases in the wound space and thus also within the sleeve-in-sleeve or cylinder-piston arrangement, pre-tensioning force of pressure spring may force the bottom wall of the cylinder-piston or sleeve-in-sleeve arrangement again against transparent wall 3020.

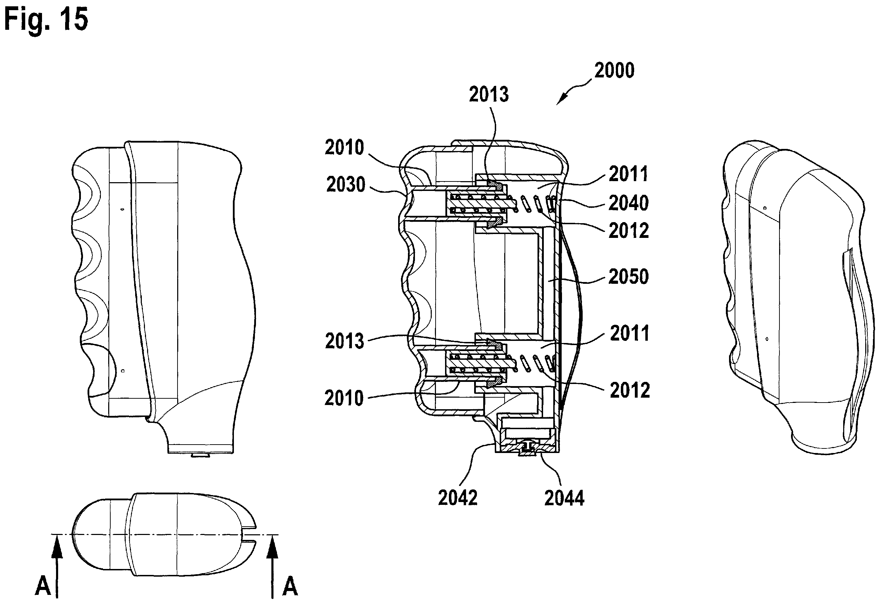

The suction device 2000 illustrated in Fig. 15 and that can be used in association with the indicator device according to Fig. 12 and 13 has a housing 2040 and a grip part 2030 that can be pushed out of the initial position illustrated in Fig. 15 into the housing. A suction head 2042, into which a one-way valve 2044 is inserted, is moulded onto the end of the housing 2040 that is shown at the bottom in Fig. 15. The suction head 2042 can be inserted into the receiver of the indicator device around which the chamber jacket 1010 passes. After introducing the suction head 2042 into the receiver, a seal system of the suction head lies against the film valve attached to the outside of the snip disc such that it passes around a recess that passes through the snap disc. In the region of the suction head the one-way valve 2044 facilitates the generation of reduced pressure in the region of the indicator device because reduced pressure can be maintained with the aid of this indicator valve by actuating the suction device between a sealing lip of the suction device 2046, which can be seen particularly clearly in Fig. 11, and the valve system made in the form of a film valve. Due to this reduced pressure, the suction device sucks on the film valve and remains adhered here, even if the suction chambers 2011 of the suction device are ventilated when actuated. In addition or alternatively to the one-way valve 2044 one can also work with a slight leakage flow.

A total of two suction chambers 2011 running parallel to one another are provided in the housing 2040. The grip part 2030 that can be inserted into the housing 2040 has two suction pistons 2010 which can be introduced into the suction chambers 2011. Upon introducing the suction pistons 2010 into the suction chambers 2011, sealing rings 2013 passing around the suction pistons 2010 are lifted from an inner boundary surface of the suction chambers 2011 and thus make it possible for gases to escape from the suction chambers 2011. By introducing the suction pistons 2010 into the suction chambers 2011, compression springs 2012 accommodated in the suction chambers 2011 are compressed. When the suction pistons 2010 are pushed onto the suction chambers 2011 by the effect of the compression springs 2012, the sealing rings 2013 rest against the inner boundary surfaces of the suction chambers 2011. By increasing the volume of the suction chambers 2011, suction is produced in suction channels 2050 which are connected to the suction head 2042. By means of the film valve on the outer boundary surface of the snap disc and the valve system 2044 in the suction head 2042, gases can thus be sucked out of the chamber of the indicator device, and so also out of the wound space, and reduced pressure is established in the chamber and in the wound space.

The invention is not restricted to the exemplary embodiments explained by means of the drawings. For example, the following modifications of the embodiment illustrated by the drawings is also conceivable:
The valve system, in the form of a film valve, and the indicator device are integrated, in combination, directly into the dressing.
The valve system and the indicator device, in combination, as illustrated by the drawings, are connected by a tube to the dressing.
The valve system and the indicator device are connected individually by means of at least one tube to the dressing.
The valve system and the indicator device are incorporated individually directly into the dressing.
The valve system is connected by a tube to the dressing, the indicator device being incorporated directly into the dressing.
The indicator device is connected by a tube to the dressing, the valve system being incorporated directly into the dressing.
The valve system and the indicator device, already pre-fabricated in combination, are applied adhesively to the dressing. For this purpose, the dressing or the covering device is provided at the appropriate point with a recess or is made to be permeable to gas.
The valve system and the indicator device, already individually pre-fabricated, are applied adhesively to the covering device. For this purpose, the covering device is respectively provided at the appropriate points with a recess and a region that is permeable to gas.

With systems according to the invention the following advantages are achieved:
A pump securely connected to the wound care system is not required. In fact, if so required, a suction device can be coupled to the valve system from the outside. After reaching the desired reduced pressure in the wound space the suction device can be removed.

Clearly improved comfort results for the user if the otherwise required connection tube to the pump can either be totally dispensed with or, instead of this connection tube or the fluid line, a clearly shortened tube to the valve system can be used.

The patient's mobility is not, as with other systems, additionally restricted by this tube. Even with a tube-bound valve system of the covering device, in comparison with a conventional system provided with a. pump, the overall system is much lighter and smaller. The risk of detachment due to inadvertent tearing on the connection tube between the covering device and the pump and associated leakage of the adhesive covering device is thus considerably reduced.

The system with the valve system and the indicator device, in combination, but also separated, is flexible due to its structure. It can be bent and buckled, and so can be worn discretely on the patient and under clothing. The patient can be informed discretely at all times of the reduced pressure status by means of the indicator device and, if need be, re-pump with the aid of a separate suction device.

The system does not require any additional energy sources such as voltage sources (e.g. battery/socket). In this way allowance is made for the defect that single-use devices with electrical voltage sources may lead to constraints relating to operability and sustainability for the users.

The length of a tube or some other connection can be made variable by using an appropriate connection element. The lack of flexibility, which restricts and possibly discourages the mobile users named as the target group, can be reduced. Even after detaching the suction device the valve system is sealed from the outside with respect to water, dust and air.

The suction device can be designed, for example, as an open piston or bellows system. Therefore, in addition to a favourable production and/or purchase price, it is insensitive to water, dust, dirt, etc. However, it can also be realised in the form of an electromechanical, mechanical, hydraulic and/or pneumatic pump.

The design of a combination of a valve system and an indicator device, as illustrated, for example, in Fig. 3, makes it possible to check the reduced pressure status on both sides, and so without any risk of confusion and, if so required, to re-pump.

By using phosphorescent (luminescent) paints or elements when producing valve systems and/or indicator devices, the reduced pressure status can also be made to be visible in the dark.

The wound care system may be designed as a "single-use product" or the suction device may be designed as a "single-patient-use product". Thus, in terms of sustainability, the amounts of consumable materials can be reduced to the greatest possible extent.

### List of reference numbers

- 1: reinforcement strip
- 2: limiting element
- 3: film
- 4: indicator layer
- 5: spacer layer
- 6: indicator layer
- 7: limiting element
- 8: tube
- 9: housing
- 10: piston
- 12: spring
- 13: sealing ring
- 14: counter-holder
- 15: valve body
- 16: sealing flange
- 17: hole
- 100: covering device
- 120: wound filling material
- 200: valve system
- 300: suction device
- 500: housing
- 530: jacket
- 600: membrane
- 640: pre-tensioning device
- 700: tube
- 701: reduced pressure-stable tube section
- 702: compressible tube section
- 703: liquid
- 704: mark
- 510,520: chambers
- 540, 550: front surface
- 620,630: sleeve
- 622, 632: hole in the front wall
- 1000: valve and indicator system
- 1005: chamber
- 1010: chamber jacket
- 1012: outer groove
- 1012a: bottom wall
- 1014: inner groove
- 1016: sealing rings
- 1020: indicator wall
- 1022: snap disc
- 1024: recess
- 1026: film valve
- 1030: monitoring wall
- 1032: upwardly curved ring
- 1035: recess
- 1040: indicator element
- 1050: channel
- 1055: transition areas
- 1055a: guiding surfaces
- 1060: annular protrusion (collar)
- 1100: suction device
- 1110: suction channel
- 1120: seal system
- 1200: tube
- 1210: stop member
- 1300: rigid support member
- 1310: abutting surface
- 1320: recess
- 1350: deformable coupling member
- 1360: circumferential profile
- 1370: coupling portion
- 1380: annular protrusion
- 2000: suction device
- 2010: suction piston
- 2011: suction chamber
- 2012: compression spring
- 2013: sealing rings
- 2030: grip part
- 2040: housing
- 2042: suction head
- 2044: one-way valve
- 2050: suction channel
- 3000: indicator chamber
- 3010: separating wall
- 3015: through hole
- 3020: feformable wall
- 3030: vircumferential jacket member
- 3100: outer jacket

## Claims

1. A wound care system for reduced pressure therapy comprising a covering device (100) that can be fixed to the skin surrounding a wound and serving to produce a closed wound space containing the wound and a valve system, (200), said valve system being assigned to the covering device, and said valve system being operable on the one hand to suck gases out of the wound space and on the other hand to counter the penetration of gases into the wound space after fixing the covering device to the skin surrounding the wound, the valve system being able to be coupled detachably to a suction device (300) that can be operated to suck gases out of the wound space, **characterised in that** the valve system comprises a film valve, the valve system has a valve body (15) with a valve chamber serving to receive gases from the wound space, and the valve chamber is approximately lens-shaped before gases are released from it and is delimited by two film-like limiting elements connected, gas-tight, to one another in the region of their edges, both of which limiting elements are provided with at least one valve slot in order to form the film valve.

2. The wound care system according to Claim 1, **characterised in that** the valve system is integrated into the covering device.

3. The wound care system according to Claim 1, **characterised in that** the valve system is coupled to the covering device by means of a fluid line, in particular a tube (8).

4. The wound care system according to any of the preceding claims, **characterised in that** the valve chamber can be compressed releasing gases held within it.

5. The wound care system according to any of the preceding claims, **characterised in that** the valve system has a one-way valve designed to discharge fluids, in particular gases, from the wound space.

6. The wound care system according to Claim 5, **characterised in that** the one-way valve has at least one valve slot formed in a preferably film-like limiting element delimiting the valve chamber.

7. The wound care system according to any preceding claim, **characterised in that** at least one limiting element has a reinforcement layer provided with a recess in the region of the at least one valve slot.

8. The wound care system according to any of Claims 3 to 7, **characterised in that** the tube coupled to the covering device leads into the valve chamber.

9. The wound care system according to any of the preceding claims, **characterised in that** the covering device is provided, at least in sections, with an adhesive agent designed to fix the covering device to the skin, preferably running around the wound space.

10. The wound care system according to any of Claims 3 to 9, **characterised in that** the fluid line is attached to the boundary surface of the covering device provided with the adhesive agent.

11. The wound care system according to any of Claims 3 to 9, **characterised in that** the fluid line passes through the covering device and/or leads into an opening that passes through the sealing device and/or the covering device has a suction port designed for coupling the fluid line.

12. The wound care system according to any of the preceding claims, **characterised in that** the valve system, in particular the valve system coupled to the covering device by means of a fluid line, has a rigid housing which preferably at least partially delimits a valve chamber and which is preferably provided with a one-way valve, in particular a film valve.

13. A wound care system according to any of the preceding claims, **characterised in that** an indicator device that can be operated to display the reduced pressure status in the wound space is assigned to the covering device.

14. The wound care system according to Claim 13, **characterised in that** the indicator device is integrated into the covering device.

## Patentansprüche

1. Wundversorgungssystem für die Vakuumtherapie, umfassend eine an der eine Wunde umgebenden Haut anbringbare Abdeckvorrichtung (100), die zum Herstellen eines geschlossenen, die Wunde enthaltenden Wundraums dient, und ein Ventilsystem (200), das der Abdeckvorrichtung zugeordnet ist, und wobei das Ventilsystem dahingehend betreibbar ist, einerseits Gase aus dem Wundraum abzusaugen und andererseits dem Eindringen von Gasen in den Wundraum nach dem Befestigen der Abdeckvorrichtung an der die Wunde umgebenden Haut entgegenzuwirken, wobei das Ventilsystem lösbar an eine Absaugvorrichtung (300) koppelbar ist, die zum Absaugen von Gasen aus dem Wundraum betreibbar ist, **dadurch gekennzeichnet, dass** das Ventilsystem ein Folienventil umfasst, das Ventilsystem einen Ventilkörper (15) mit einer zur Aufnahme von Gasen aus dem Wundraum dienenden Ventilkammer aufweist und die Ventilkammer vor der Freigabe von Gasen aus ihr etwa linsenförmig ist und von zwei folienartigen Begrenzungselementen begrenzt ist, die im Bereich ihrer Ränder gasdicht miteinander verbunden sind, wobei beide Begrenzungselemente zur Bildung des Folienventils mit mindestens einem Ventilschlitz versehen sind.

2. Wundversorgungssystem nach Anspruch 1, **dadurch gekennzeichnet, dass** das Ventilsystem in die Abdeckvorrichtung integriert ist.

3. Wundversorgungssystem nach Anspruch 1, **dadurch gekennzeichnet, dass** das Ventilsystem über eine Fluidleitung, insbesondere einen Schlauch (8), an die Abdeckvorrichtung gekoppelt ist.

4. Wundversorgungssystem nach einem der vorhergehenden Ansprüche, **dadurch gekennzeichnet, dass** die Ventilkammer komprimierbar ist und darin gehaltene Gase freigibt.

5. Wundversorgungssystem nach einem der vorhergehenden Ansprüche, **dadurch gekennzeichnet, dass** das Ventilsystem ein Einwegventil aufweist, das zum Abführen von Fluiden, insbesondere Gasen, aus dem Wundraum ausgebildet ist.

6. Wundversorgungssystem nach Anspruch 5, **dadurch gekennzeichnet, dass** das Einwegventil mindestens einen Ventilschlitz aufweist, der in einem die Ventilkammer begrenzenden, vorzugsweise folienartigen Begrenzungselement ausgebildet ist.

7. Wundversorgungssystem nach einem der vorhergehenden Ansprüche, **dadurch gekennzeichnet, dass** mindestens ein Begrenzungselement eine Verstärkungsschicht aufweist, die im Bereich des mindestens einen Ventilschlitzes mit einer Aussparung versehen ist.

8. Wundversorgungssystem nach einem der Ansprüche 3 bis 7, **dadurch gekennzeichnet, dass** der an die Abdeckvorrichtung gekoppelte Schlauch in die Ventilkammer führt.

9. Wundversorgungssystem nach einem der vorhergehenden Ansprüche, **dadurch gekennzeichnet, dass** die Abdeckvorrichtung mindestens abschnittsweise mit einem Haftmittel versehen ist, das zur Fixierung der Abdeckvorrichtung auf der Haut, vorzugsweise umlaufend um den Wundraum, ausgebildet ist.

10. Wundversorgungssystem nach einem der Ansprüche 3 bis 9, **dadurch gekennzeichnet, dass** die Fluidleitung an der mit dem Haftmittel versehenen Grenzfläche der Abdeckvorrichtung angebracht ist.

11. Wundversorgungssystem nach einem der Ansprüche 3 bis 9, **dadurch gekennzeichnet, dass** die Fluidleitung durch die Abdeckvorrichtung geht und/oder in eine Öffnung mündet, die durch die Abdichtvorrichtung geht und/oder die Abdeckvorrichtung einen zur Koppelung der Fluidleitung ausgebildeten Ansaugstutzen aufweist.

12. Wundversorgungssystem nach einem der vorhergehenden Ansprüche, **dadurch gekennzeichnet, dass** das Ventilsystem, insbesondere das über eine Fluidleitung an die Abdeckvorrichtung gekoppelte Ventilsystem, ein starres Gehäuse aufweist, das vorzugsweise mindestens teilweise eine Ventilkammer begrenzt und das vorzugsweise mit einem Einwegventil, insbesondere einem Folienventil, versehen ist.

13. Wundversorgungssystem nach einem der vorhergehenden Ansprüche, **dadurch gekennzeichnet, dass** der Abdeckvorrichtung eine Anzeigevorrichtung zugeordnet ist, die zur Anzeige des druckreduzierten Zustands in dem Wundraum betätigbar ist.

14. Wundversorgungssystem nach Anspruch 13, **dadurch gekennzeichnet, dass** die Anzeigevorrichtung in die Abdeckvorrichtung integriert ist.

## Revendications

1. Système de soin de plaie pour thérapie par pression réduite comprenant un dispositif de recouvrement (100) pouvant être fixé à la peau entourant une plaie et servant à créer un espace fermé de plaie contenant la plaie et un système de soupape, (200), ledit système de soupape étant attribué au dispositif de recouvrement, et ledit système de soupape étant exploitable d'une part pour aspirer des gaz hors de l'espace de plaie et d'autre part pour empêcher la pénétration de gaz dans l'espace de plaie après la fixation du dispositif de recouvrement à la peau entourant la plaie, le système de soupape pouvant être accouplé de manière amovible à un dispositif d'aspiration (300) pouvant être actionné pour aspirer des gaz hors de l'espace de plaie, **caractérisé en ce que** le système de soupape comprend une soupape à membrane, le système de soupape a un corps de soupape (15) pourvu d'une chambre de soupape servant à recevoir des gaz provenant de l'espace de plaie, et la chambre de soupape a approximativement la forme d'une lentille avant que les gaz ne soient évacués de celle-ci et est délimitée par deux éléments de délimitation de type membrane reliés entre eux de manière étanche aux gaz dans la région de leurs bords, les deux éléments de délimitation étant pourvus d'au moins une fente de soupape pour former la soupape à membrane.

2. Système de soin de plaie selon la revendication 1, **caractérisé en ce que** le système de soupape est intégré dans le dispositif de recouvrement.

3. Système de soin de plaie selon la revendication 1, **caractérisé en ce que** le système de soupape est accouplé au dispositif de recouvrement au moyen d'une ligne de fluide, en particulier un tube (8).

4. Système de soin de plaie selon l'une quelconque des revendications précédentes, **caractérisé en ce que** la chambre de soupape peut être comprimée pour libérer les gaz contenus en son sein.

5. Système de soin de plaie selon l'une quelconque des revendications précédentes, **caractérisé en ce que** le système de soupape a une soupape unidirectionnelle conçue pour évacuer des fluides, en particulier des gaz, de l'espace de plaie.

6. Système de soin de plaie selon la revendication 5, **caractérisé en ce que** la soupape unidirectionnelle a au moins une fente de soupape formée dans un élément de limitation de préférence de type membrane délimitant la chambre de soupape.

7. Système de soin de plaie selon l'une quelconque revendication précédente, **caractérisé en ce qu'**au moins un élément de limitation a une couche de renfort pourvue d'un évidement dans la région de l'au moins une fente de soupape.

8. Système de soin de plaie selon l'une quelconque des revendications 3 à 7, **caractérisé en ce que** le tube accouplé au dispositif de recouvrement débouche dans la chambre de soupape.

9. Système de soin de plaie selon l'une quelconque des revendications précédentes, **caractérisé en ce que** le dispositif de recouvrement est pourvu, au moins dans certaines sections, d'un agent adhésif conçu pour fixer le dispositif de recouvrement à la peau, de préférence en contournant l'espace de plaie.

10. Système de soin de plaie selon l'une quelconque des revendications 3 à 9, **caractérisé en ce que** la ligne de fluide est fixée à la surface limite du dispositif de recouvrement pourvue de l'agent adhésif.

11. Système de soin de plaie selon l'une quelconque des revendications 3 à 9, **caractérisé en ce que** la ligne de fluide traverse le dispositif de recouvrement et/ou débouche dans une ouverture qui traverse le dispositif d'étanchéité et/ou le dispositif de recouvrement a un orifice d'aspiration conçu pour l'accouplement de la ligne de fluide.

12. Système de soin de plaie selon l'une quelconque des revendications précédentes, **caractérisé en ce que** le système de soupape, en particulier le système de soupape accouplé au dispositif de recouvrement au moyen d'une ligne de fluide, a un boîtier rigide qui délimite de préférence au moins partiellement une chambre de soupape et qui est de préférence pourvu d'une soupape unidirectionnelle, en particulier d'une soupape à membrane.

13. Système de soin de plaie selon l'une quelconque des revendications précédentes, **caractérisé en ce qu'**un dispositif indicateur pouvant être actionné pour afficher l'état de pression réduite dans l'espace de la plaie est associé au dispositif de recouvrement.

14. Système de soin de plaie selon la revendication 13, **caractérisé en ce que** le dispositif indicateur est intégré dans le dispositif de recouvrement.
